(19) Europäisches Patentamt
European Patent Office
Office européen des brevets

(11) EP 4 424 699 A1

(12) EUROPEAN PATENT APPLICATION

(43) Date of publication:
**04.09.2024 Bulletin 2024/36**

(21) Application number: **23159984.6**

(22) Date of filing: **03.03.2023**

(51) International Patent Classification (IPC):
***C07K 7/06*** *(2006.01)* ***C07K 7/08*** *(2006.01)*
***C07K 14/005*** *(2006.01)*

(52) Cooperative Patent Classification (CPC):
**C07K 7/06; A61P 1/16; A61P 31/20; C07K 7/08; C07K 14/005;** A61K 38/00; C07K 2319/10; C12N 2730/10122; C12N 2730/10133

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Julius-Maximilians-Universität Würzburg**
**97070 Würzburg (DE)**

(72) Inventor: **The designation of the inventor has not yet been filed**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

# (54) HBV CORE PROTEIN BINDING COMPOUNDS AND USES THEREOF

(57) The present invention relates to a compound comprising a multimer, preferably a dimer, of peptides or peptide analogues each comprising the amino acid sequence $X_1X_2LGX_3X_4$ and a linker connecting the peptides or peptide analogues, and pharmaceutical compositions and kits comprising said compound. The present invention further relates to the compound for use in the treatment of a Hepatitis B virus (HBV) infection or a liver disease caused by HBV. The present invention also relates to a method of the treatment of a HBV infection or a liver disease caused by HBV.

EP 4 424 699 A1

## Description

The present invention relates to a compound comprising a multimer, preferably a dimer, of peptides or peptide analogues each comprising the amino acid sequence $X_1X_2LGX_3X_4$ and a linker connecting the peptides or peptide analogues, and pharmaceutical compositions and kits comprising said compound. The present invention further relates to the compound for use in the treatment of a Hepatitis B virus (HBV) infection or a liver disease caused by HBV. The present invention also relates to a method of the treatment of a HBV infection or a liver disease caused by HBV.

BACKGROUND OF THE INVENTION

Hepatitis B virus (HBV) is a major human pathogen from the *Hepadnaviridae* family that consists of an outer lipid envelope and an icosahedral protein nucleocapsid core that encapsulates partially double-stranded DNA. Despite the worldwide vaccination program, HBV remains a global health problem. In 2019, at least 296 million people, or 4% of the world's population, had chronic Hepatitis B. HBV infection is the main cause of liver cirrhosis and hepatocellular carcinoma, estimated to be responsible for ~800,000 deaths annually (Block et al., 2021; World Health Organisation, 2022). In 2016, the World Health Organization set a goal of eliminating viral hepatitis as a public health threat by 2030. Achieving this goal requires the development of novel therapeutic treatments to cure chronic hepatitis B, medications that will prevent its transmission and expanding the vaccination program to prevent new infections (Block et al., 2021; Cox et al., 2020).

For HBV, so far, the available therapies include nucleoside/nucleotide analogues and alpha-interferons, which are effective in reducing the viral load, but are poorly effective in curing persistent infection (Block et al., 2021; Hu et al., 2019). However over 50 drugs are currently in development, among them 13 capsid inhibitors, as can be seen on the Website of the Hepatitis B Foundation (https://www.hepb.org/treatment-and-management/drug-watch/).

There is a need in the art for improved means and methods for treating HBV infections as well as chronic hepatitis B.

SUMMARY OF THE INVENTION

According to the present invention this object is solved by a compound comprising

(i) a multimer of a peptide or peptide analogue **P,**
wherein the peptide or peptide analogue **P** comprises the amino acid sequence

$\mathbf{X_1X_2LGX_3X_4}$ SEQ ID NO. 1,

wherein

- $\mathbf{X_1}$ is selected from serine (S), glutamine (Q), asparagine (N), glycine (G), glutamate (E), aspartate (D), alanine (A), and non-natural amino acids comprising hydrophilic exchange(s),
- $\mathbf{X_2}$ is selected from leucine (L), isoleucine (I) and non-natural amino acids comprising hydrophobic exchange(s),
- $\mathbf{X_3}$ is selected from arginine (R), phenylalanine (F), glutamic acid (E) and non-natural amino acids comprising hydrophobic or hydrophilic exchange(s),
- $\mathbf{X_4}$ is selected from methionine (M), valine (V), leucine (L), isoleucine (I) phenylalanine (F), and non-natural amino acids comprising hydrophobic exchange(s), and wherein the peptide or peptide analogue has a length of 6 to 13 amino acids,

and
(ii) a linker **L** connecting the peptides or peptide analogues **P,**
wherein the linker has a length of 60 to 160 Å.

According to the present invention this object is solved by a pharmaceutical composition comprising

a compound of the present invention, and
optionally, pharmaceutically acceptable excipient(s) and/or carrier.

According to the present invention this object is solved by using a compound of the present invention for binding and/or aggregating the core protein of Hepatitis B virus (HBV).

According to the present invention this object is solved by providing the compound of the present invention or

the pharmaceutical composition of the present invention for use in medicine.

According to the present invention this object is solved by providing the compound of the present invention or the pharmaceutical composition of the present invention for use in the treatment of a Hepatitis B virus (HBV) infection or a liver disease caused by HBV.

According to the present invention this object is solved by a kit comprising

(a) a compound of the present invention or a pharmaceutical composition of the present invention, and
(b) a cell penetrating peptide (CPP), preferably a thiol-reactive CPP.

According to the present invention this object is solved by a method for the treatment of a Hepatitis B virus (HBV) infection or a liver disease caused by HBV, comprising the administration of a therapeutically effective amount of a compound of the present invention or the pharmaceutical composition of the present invention to a subject in need thereof.

DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "6 to 13" should be interpreted to include not only the explicitly recited values of 6 to 13, but also include individual values and sub-ranges within the indicated range. Thus, included in this numerical range are individual values such as 6, 7, 8, 9, 10, 11, 12, 13 and sub-ranges such as from 6 to 12, 6 to 10, etc. This same principle applies to ranges reciting only one numerical value, such as "at least 6". Furthermore, such an interpretation should apply regardless of the breadth of the range or the characteristics being described.

*Compounds binding to the HBV core protein*

The present inventors have developed compounds that bind and/or aggregate the core protein of Hepatitis B virus.

A compound according to the present invention comprises

(i) a multimer of a peptide or peptide analogue **P,** and
(ii) a linker **L** connecting the peptides.

*(i) Multimer of peptides*

The compounds of the present invention comprise a multimer of peptides or peptide analogues **P.**

The multimer can be a dimer, trimer, tetramer, pentamer or comprise more than five peptides or peptide analogues P.

A "peptide analogue" as used herein refers to a modified peptide or to a compound that mimics structural or functional aspects of a peptide. The term "peptidomimetic" can be used interchangeably with the term "peptide analogue". Examples are D-peptides, $\beta$-peptides or peptoids. In peptoids, the side chain is connected to the nitrogen of the peptide backbone, instead of the $\alpha$-carbon as in peptides. The altered chemical structure of a peptide analogue or peptidomimetic is designed to advantageously adjust the molecular properties, such as stability or biological activity while maintaining structural or functional features. Peptide analogues or peptidomimetics can also comprise non-natural amino acids. These modifications involve changes to the peptide that will not occur naturally, such as altered backbones or altered sidechains. Non-natural amino acids can be generated from their native analogs via modifications, such as amine alkylation, side chain substitution, structural bond extension cyclization, and isosteric replacements within the amino acid backbone.

The peptide or peptide analogue P comprises the amino acid sequence

$X_1X_2LGX_3X_4$ (SEQ ID NO. 1),

wherein:

- $X_1$ is selected from serine (S), glutamine (Q), asparagine (N), glycine (G), glutamate (E), aspartate (D) and alanine (A), and non-natural amino acids comprising hydrophilic exchange(s),
  such as D-serine (s), D-glutamine (q), D-asparagine (n), D-glutamate (e), D-aspartate (d) and D-alanine (a), and further amino acid side chains with a carboxyl group in their side chain.
- $X_2$ is selected from leucine (L), isoleucine (I) and non-natural amino acids comprising hydrophobic exchange(s), such as 3-aminopentane-3-carboxylic acid, L-cyclopentylglycine, L-cyclopropylglycine, L-cyclohexylglycine, L-t-leucine, L-neopentylglycine, alpha-methyl-L-valine, and allylglycine.
- $X_3$ is selected from arginine (R), phenylalanine (F), glutamic acid (E) and non-natural amino acids comprising hydrophobic or hydrophilic exchange(s),
  such as L-phenylglycine, and L-Tyr or L-Phe substituted with halogene (Br, J or Cl).
- $X_4$ is selected from methionine (M), valine (V), leucine (L), isoleucine (I), phenylalanine (F), and non-natural amino acids comprising hydrophobic exchange(s),
  such as 3-aminopentane-3-carboxylic acid, L-cyclopentylglycine, L-cyclopropylglycine, L-cyclohexylglycine, L-t-leucine, L-neopentylglycine, alpha-methyl-L-valine, allylglycine, L-phenylglycine, and L-Tyr or L-Phe substituted with halogene (Br, J or Cl).

In a preferred embodiment,

- $X_1$ is selected from serine (S), glutamine (Q), asparagine (N), glycine (G), glutamate (E), aspartate (D) and alanine (A),
- $X_2$ is selected from leucine (L) and isoleucine (I),
- $X_3$ is selected from arginine (R), phenylalanine (F), and glutamic acid (E), preferably arginine (R) and phenylalanine (F),
- $X_4$ is selected from methionine (M), valine (V), leucine (L), isoleucine (I) and phenylalanine (F).

The peptides or peptide analogues of the present invention comprise amino acids, which can be both L- or D-amino acids.

In one embodiment, the amino acid in position $X_1$ is a non-natural amino acid comprising a hydrophilic exchange. Examples for such non-natural amino acids comprising hydrophilic exchange(s) include but are not limited to D-serine (s), D-glutamine (q), D-asparagine (n), D-glutamate (e), D-aspartate (d) and D-alanine (a), and further amino acid side chains with a carboxyl group in their side chain.

In one embodiment, the amino acid in position $X_2$ and/or $X_4$ is a non-natural amino acid comprising a hydrophobic exchange.

Examples for such non-natural amino acids comprising hydrophobic exchange(s) include but are not limited to 3-aminopentane-3-carboxylic acid, L-cyclopentylglycine, L-cyclopropylglycine, L-cyclohexylglycine, L-t-leucine, L-neopentylglycine, alpha-methyl-L-valine, allylglycine, L-phenylglycine and L-Tyr or L-Phe substituted with halogene (Br, J or Cl).

In one embodiment, the amino acid in position $X_3$ is a non-natural amino acid comprising a hydrophobic exchange or a hydrophilic exchange.

- $X_1$ is preferably serine (S).
- $X_2$ is preferably leucine (L).
- $X_3$ is preferably arginine (R),
- $X_4$ is preferably leucine (L) or methionine (M).

In one embodiment, $X_1$ is serine (S), $X_2$ is leucine (L), $X_3$ is arginine (R) and/or $X_4$ is leucine (L) or methionine (M).

In one embodiment, P comprises or consists of the amino acid sequence SLLGR$X_4$ (SEQ ID NO. 2), wherein $X_4$ is selected from methionine (M), valine (V), leucine (L), isoleucine (I), phenylalanine (F), and non-natural amino acids comprising hydrophobic exchange(s), as defined above, preferably from M, L, I, V or F. $X_4$ is preferably L or M.

Said amino acid sequence of SEQ ID NO. 2 is the core binding motif or essential sequence for binding to the HBV core protein.

According to the invention, the compound of the present invention comprises at least two of said core binding motifs or essential sequences.

The peptide or peptide analogue P has a length of at least 6 amino acids.

The length is in the range of 6 to 13 amino acids, preferably 6 to 12 amino acids, such as 6 amino acids, 10 amino acids or 12 amino acids.

In a preferred embodiment, the peptide or peptide analogue P has a length of 10 amino acids

In one embodiment, the peptide or peptide analogue P comprises or consists of the amino acid sequence of GSLLGR$\mathbf{X_4}$KGA (SEQ ID NO. 3) or MHRSLLGR$\mathbf{X_4}$KGA (SEQ ID NO. 4), wherein $\mathbf{X_4}$ is selected from methionine (M), valine (V), leucine (L), isoleucine (I), phenylalanine (F), and non-natural amino acids comprising hydrophobic exchange(s), as defined above, preferably from M, L, I, V or F.

$\mathbf{X_4}$ is preferably L or M.

In one embodiment, the peptide or peptide analogue P comprises or consists of the amino acid sequence of

| | |
|---|---|
| SLLGR$\mathbf{X_4}$ | SEQ ID NO. 2, |
| GSLLGR$\mathbf{X_4}$KGA | SEQ ID NO. 3, or |
| MHRSLLGR$\mathbf{X_4}$KGA | SEQ ID NO. 4, |

wherein $\mathbf{X_4}$ is selected from methionine (M), valine (V), leucine (L), isoleucine (I), phenylalanine (F), and non-natural amino acids comprising hydrophobic exchange(s), as defined above, preferably from M, L, I, V or F, as defined above.

$\mathbf{X_4}$ is preferably L or M.

In one embodiment, the peptide or peptide analogue P comprises or consists of an amino acid sequence which is selected from

| | |
|---|---|
| SLLGRM | SEQ ID NO. 5, |
| SLLGRL | SEQ ID NO. 6, |
| SLLGRI | SEQ ID NO. 7, |
| SLLGRV | SEQ ID NO. 8, |
| SLLGRF | SEQ ID NO. 9, |
| GSLLGRMKGA | SEQ ID NO. 10, |
| GSLLGRLKGA | SEQ ID NO. 11, |
| GSLLGRIKGA | SEQ ID NO. 12, |
| GSLLGRVKGA | SEQ ID NO. 13, |
| GSLLGRFKGA | SEQ ID NO. 14, |
| MHRSLLGRMKGA | SEQ ID NO. 15, |
| MHRSLLGRLKGA | SEQ ID NO. 16, |
| MHRSLLGRIKGA | SEQ ID NO. 17, |
| MHRSLLGRVKGA | SEQ ID NO. 18, and |
| MHRSLLGRFKGA | SEQ ID NO. 19, |

more preferably

| | |
|---|---|
| SLLGRM | SEQ ID NO. 5, |
| SLLGRL | SEQ ID NO. 6, |
| GSLLGRMKGA | SEQ ID NO. 10, |
| GSLLGRLKGA | SEQ ID NO. 11, |
| MHRSLLGRMKGA | SEQ ID NO. 15, and |
| MHRSLLGRLKGA | SEQ ID NO. 16. |

In a preferred embodiment, the compound of the present invention has formula I

$$\mathbf{(P)_m} - \mathrm{L} - \mathbf{(Z)_n} \qquad \text{(I)},$$

wherein

**P** is the peptide or peptide analogue,
**m** is at least 2, preferably, 2, 3, 4 or 5,
**L** is the linker connecting the peptides or peptide analogues **P,**
**Z** is a further moiety and **n** is 0 or 1.

In one embodiment, m is 2. The compound has formula Ia:

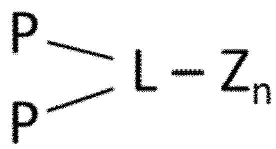

(Ia).

The two peptides or peptide analogues P can be identical in their amino acid sequence or can have a different amino acid sequence.

The two peptides or peptide analogues P can have the same length or have a different length. The dimer is considered a homodimer, when the two peptides or peptide analogues are identical in their amino acid sequence and their length.

The dimer is considered a heterodimer, when the two peptides or peptide analogues differ in their amino acid sequence or their length or both.

In one embodiment, m is 3. The compound has formula Ib:

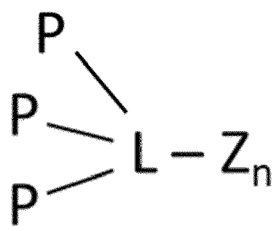

(Ib).

The three peptides or peptide analogues P can be identical in their amino acid sequence or can have a different amino acid sequence.

The three peptides or peptide analogues P can have the same length or have a different length.

In one embodiment, m is 4. The compound has formula Ic:

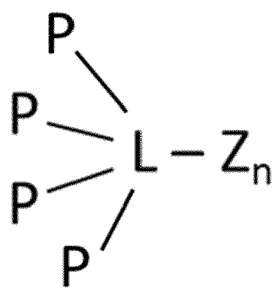

(Ic).

The four peptides or peptide analogues P can be identical in their amino acid sequence or can have a different

amino acid sequence.

The four peptides or peptide analogues P can have the same length or have a different length.

In one embodiment, m is 5. The compound has formula Id:

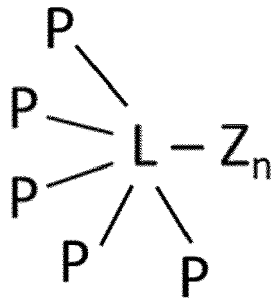

(Id).

The five peptides or peptide analogues P can be identical in their amino acid sequence or can have a different amino acid sequence.

The five peptides or peptide analogues P can have the same length or have a different length.

*(ii) Linker*

The compounds of the present invention comprise a linker **L** which connects the peptides or peptide analogues **P.**

The linker has a length of 60 to 160 Angstrom (Å), preferably 60 to 100 Å.

In a preferred embodiment the linker has a length of 80 Å.

The linker may be covalently bound to the peptides or peptide analogues simultaneously. Said linker may be bound to the C-terminus of each peptide or peptide analogue. In one embodiment, the linker is bound to a cysteine situated at the C-terminal end of each peptide or peptide analogue. The binding may be via the sulfur atom comprised in the cysteine. The said linker may be attached to the C-terminal end of each peptide or peptide analogue, for example via an amide bond with the terminal amino acid.

The linker keeps the peptides or peptide analogues comprised in the multimer, such as the dimer, at a certain distance from each other, so that they can interact with several (such as two) binding sites simultaneously, hence enhancing the affinity of the compound of the invention. In a preferred embodiment, the several (such as two) binding sites are located on different HBV core particles (such as two different HBV core particles).

The linker brings the peptides or peptide analogues P into a preferred or suitable proximity to induce aggregation.

The linker may keep the peptides or peptide analogues comprised in the multimer, such as the dimer, at a distance between 60 and 160 Å from each other. The distance may be at least 60 Å. The distance may also be at least 70 Å. The distance may also be at least 80 Å. The distance may also be at least 90 Å. The distance may also be at least 100 Å. The distance may also be about 90 Å. The distance may also be about 100 Å. The distance may also be about 110 Å. The distance may also be about 120 Å. A preferred distance is about 80 Å.

Preferably, the linker **L** comprises or consists of

an alkane chain,
diaminoacetic acid,
maleimide,
ethylene glycol,
polyethylene glycol (PEG),
*N*PEG,
glycine,
polyglycine, or
any combination thereof.

In a preferred embodiment, the linker **L** comprises at least two ethylene glycol moieties, such as 12, and/or at least two glycine,
and/or polyglycine.

In one embodiment, the linker **L** comprises at least 12 ethylene glycol moieties which results in a distance of at least 60 Å between the two peptides.

The term "ethylene glycol moiety", also called oxyethylene, as used herein refers to the structural unit that constitutes a polyethylene glycol (PEG).

The term "*N*PEG ", is a linker derived from the classical PEG, but where one or more of the backbone oxygen atoms is replaced with a nitrogen atom.

In one embodiment, the linker **L** comprises the following structure

- $[-NH-(CH_2)_2-O-(CH_2)_2-O-(CO)-]_4-NH-(CH_2)_4-CH(R^1)-NH-[-(CO)-CH_2-O-(CH_2)_2-O-(CH_2)_2-NH]_4-$.
- *further moiety*

In a preferred embodiment, the compound of the present invention comprises a further moiety **Z** which is connected to the linker L.

In one embodiment, the further moiety Z is a reactive group.

Said reactive group is for binding to a cell-penetrating peptide (CPP).

A cell penetrating peptide (CPP) as used herein refers to a peptide characterized by the ability to cross the plasma membrane of mammalian cells, and thereby may give rise to the intracellular delivery of cargo molecules, such as peptides, proteins, oligonucleotides to which it is linked.

The CPP preferably comprises at least 4 and at the most 13 amino acid residues selected from arginine and/or lysine. In one embodiment, the CPP comprises 5 arginine residues. In one embodiment, the CPP comprises 10 arginine residues.

In one embodiment, the reactive group is a thiol group. In the embodiment, where the reactive group is a thiol group, the CPP is a thiol-reactive CPP.

An example for a thiol-reactive CPP is TNB-R10, which is a CPP containing 10 arginine residues and a cysteine bound to 5-thio-2-nitro-benzoic acid for reacting with the thiol group:

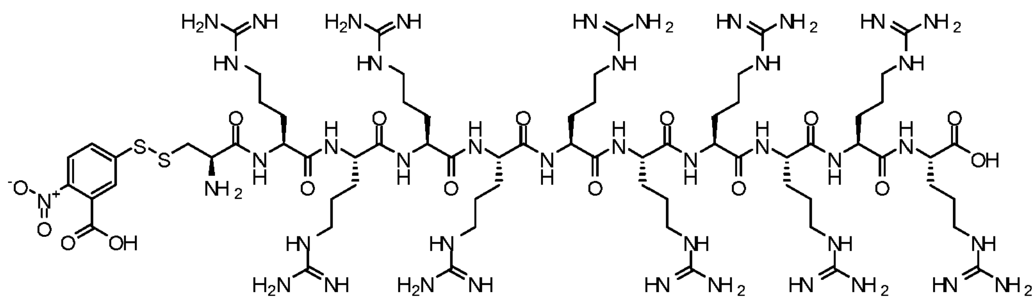

In one embodiment, the further moiety Z is a moiety comprising said reactive group.

In the embodiment, where the reactive group is a thiol group, said moiety comprising the thiol group is cysteine, homocysteine, 2-amino-5-mercaptopentanoic acid, alpha-methyl-cysteine, penicillamine, or 5-mercapto-norvaline, preferably cysteine.

In one embodiment, where the reactive group is a thiol group, the CPP is a thiol-reactive CPP, as discussed above.

In a preferred embodiment the linker has a compound comprising a thiol group as further moiety Z attached as follows:

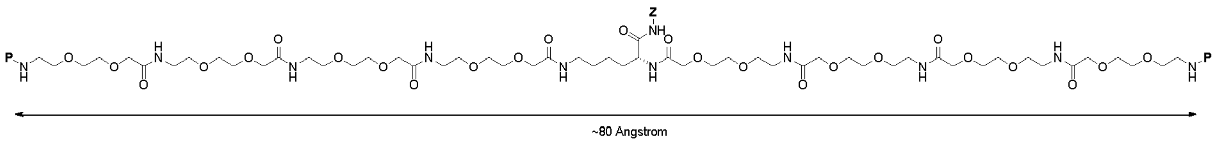


See also Figure 2E.

In one embodiment, the further moiety Z is a CPP.

In this embodiment, the CPP is directly bound to the linker, such as via an amide bond.

*Pharmaceutical composition, kit and use*

As outlined above, the present invention provides a pharmaceutical composition comprising a compound of the present invention.

A pharmaceutical composition comprising a compound of the present invention optionally comprises one or more pharmaceutically acceptable excipients and/or carrier.

Preferably, the pharmaceutical composition is suitable for intravenous, subcutaneous, intranasal, buccal, sublingual, oral, rectal and/or intraperitoneal administration.

As outlined above, the present invention provides a kit comprising

(a) a compound of the present invention or a pharmaceutical composition of the present invention, and
(b) a cell-penetrating peptide (CPP).

In a preferred embodiment, the CPP is a thiol-reactive CPP, as discussed above.

As outlined above, the present invention provides the use of a compound of the present invention for binding and/or aggregating the core protein of Hepatitis B virus (HBV).

Said binding and/or aggregating of the HBV core protein is for preventing the virion formation and/or reducing the viral load.

*Medical uses*

As outlined above, the present invention provides a compound of the present invention or a pharmaceutical composition of the present invention for use in medicine.

As outlined above, the present invention provides a compound of the present invention or a pharmaceutical composition of the present invention for use in the treatment of a Hepatitis B virus (HBV) infection or a liver disease caused by HBV.

In one embodiment, the HBV infection is a chronic HBV infection.

In one embodiment, the liver disease caused by HBV is hepatitis.

In one embodiment, the use comprises the use of a CPP.

In a preferred embodiment, the CPP is a thiol-reactive CPP, as discussed above.

*Treatment methods*

As outlined above, the present invention provides a method for the treatment of a Hepatitis B virus (HBV) infection or a liver disease caused by HBV.

Said method comprises the administration of a therapeutically effective amount of a compound of the present invention or the pharmaceutical composition of the present invention to a subject in need thereof.

A "therapeutically effective amount" of a compound of the present invention refers to the amount which has to be administered to a subject in need thereof in order to achieve a desired therapeutic result or outcome. The skilled artisan will be able to determine said therapeutically effective amount and the suitable administration regimen.

In one embodiment, the HBV infection is a chronic HBV infection.

In one embodiment, the liver disease caused by HBV is hepatitis.

In one embodiment, the method comprises the co-administration of a cell penetrating peptide (CPP).

In a preferred embodiment, the CPP is a thiol-reactive CPP, as discussed above.

The administration of the compound or the pharmaceutical composition is preferably intravenous, subcutaneous, intranasal, buccal, sublingual, oral, rectal and intraperitoneal.

*Preferred embodiments*

Hepatitis B is a global public health threat with hundreds of millions of people affected worldwide. Hepatitis B virus is a cause of this disease. The available therapy has low efficacy in curing persistent infection; therefore, an exhaustive effort is done to bring new therapeutics to the market.

We have developed a peptidic compound capable of affecting the core protein of the virus, that encircles the viral DNA. We invented a synthetic HBc binding dimeric peptidic superbinder, that has nanomolar affinity and induces core protein aggregation *in vitro* and evidently in live cells expressing the recombinant HBV core protein HBc. Our novel HBc aggregator has great potential as lead substance for the interference with the envelopment of the nucleocapsid.

*- Introduction*

The hepatotropic hepatitis B virus (HBV) and its satellite virus hepatitis D (HDV) infect the liver (Watashi & Wakita, 2015). An infection with HBV is usually cleared from the body with lifelong immunity but a significant number of infections become chronic. Chronic hepatitis can cause severe complications including steatosis, cirrhosis and hepatocellular carcinoma (HCC). Despite the existence of a vaccine which efficiently protects against HBV, with 296 million chronic carriers and over 800 000 of hepatitis-related yearly deaths, the disease remains a global health problem (Block et al., 2021; World Health Organisation, 2022).

HBV consists of a lipid bilayer with surface proteins that envelopes a capsid, a multimer assembled from HBc proteins, that contains the viral DNA (Figure 1). The virus uses the surface lipopeptide pre-S1 for docking to mature liver cells via the hepatocyte sodium/bile acid cotransporter (sodium taurocholate co-transporting polypeptide - NTCP) and subsequently entering the cells (Tian *et al.,* 2021; Watashi & Wakita, 2015). A recently EMA-approved peptide-based drug Hepcludex is based on the viral pre-S1 and also binds NTCP, thereby blocking the viral entry to hepatocytes (Gripon *et al.,* 2005; Volz *et al.,* 2013).

More promising peptides were identified in a screen of a phage library. The peptide LLGRMK (SEQ ID NO. 20) bound the full-length wild type HBc (fl wt HBc) and the related octa-peptide ALLGRMKG (SEQ ID NO. 21) inhibited HBc's interaction to the surface protein L-HBs (Dyson & Murray, 1995). In a subsequent study, using Cryo-EM and image processing, the peptide GSLLGRMKGA (SEQ ID NO. 10) was seen to bind the tips of the spikes of recombinantly purified HBc capsids(Böttcher et al., 1998). Additionally, the same study demonstrated that GSLLGRMKGA (SEQ ID NO. 10) and the related peptides SLLGRMKGA (SEQ ID NO. 22) and SLLGRMKG (SEQ ID NO. 23) were able to inhibit HBV production in Hep G2 human hepatoblastoma cell lines with IC50 values of 0.8, 2.4 and 6.4 μM, respectively. In a recent study, we confirmed that the tips of capsid spikes are the binding sites for the peptides MHRSLLGRMKGA (Pep1) (SEQ ID NO. 15) and GSLLGRMKGA (Pep2) (SEQ ID NO. 10) with $K_D$ values of 26 and 68 μM, respectively (Makbul, Khayenko, *et al.,* 2021). In the same study we identified SLLGRM (SEQ ID NO. 5) as the shortest peptide still able to bind recombinant HBc capsids with a $K_D$ value of 130 μM. However, the relatively low affinity and inability to effectively penetrate the cell membrane make these peptides poor pharmacological agents.

Based on GSLLGRMKGA, MHRSLLGRMKGA and SLLGRM (SEQ ID NOs. 15, 10 and 5), we designed and synthesized peptidic dimers that bind HBc capsids with low micro- to nanomolar affinity and aggregate them in vitro. More importantly, in combination with thiol-reactive cell penetrating peptides, our MHRSLLGRMKGA-based thiolated dimeric superbinder P1dC, induces HBc core protein aggregation in live mammalian cells expressing recombinant HBV core protein. These new core protein aggregating peptidic dimers have great potential as new lead compounds for the interference with the DNA protein shield of HBV.

*- Results*

The peptides SLLGRM, GSLLGRMKGA and MHRSLLGRMKGA (SEQ ID NOs. 5, 15 and 10) bind to wt and mutant HBc variants with micromolar affinity (Makbul, Khayenko, *et al.,* 2021). To increase the affinity of these peptides, we used the concept of avidity that is exceptionally useful in the case of HBc capsids, with 90 or 120 potential binding sites. We synthesised dimeric versions of SLLGRM, GSLLGRMKGA and MHRSLLGRMKGA (SEQ ID NOs. 5, 15 and 10) and connected them with PEG linkers (Figure 2).

The tips of the capsid spikes are spaced about 6 nm apart, so a simple dimer (as for example SLLGRM-SLLGRM) would not be long enough to simultaneously bind two capsid spikes. Therefore, the linker which connects the two peptides must be long enough to bridge the capsid spikes which are spaced about 6 nm apart. Additionally, the linker should be flexible so the dimers can assume a multitude of different conformations (Figure 2E).

Using isothermal titration calorimetry (ITC), we first determined that SLLGRM-, GSLLGRMKGA-, and MHRSLLGRMKGA-dimers bind the HBc capsids (Figure 3). Gratifyingly, the $K_D$ values of 4.89, 1.91 and 0.312 μM of the dimers, were a 26-, 35- and 83-fold affinity increase over the respective monomers (130, 68 and 26 μM) (Makbul, Khayenko, *et al.,* 2021) (Table 1).

**Table 1** Summary of thermodynamic parameters obtained by ITC experiments using the peptide dimers and fl wt HBc capsids. In case of the Pep2-dimer the deviations represent deviations of fit since only one ITC experiment was performed. N represents the stoichiometry.

| Peptide [SEQ ID NO. of monomer] | N | $K_a$ $\frac{1}{M}$ | $K_D$ μM | ΔH $\frac{cal}{mol}$ | ΔS $\frac{cal}{mol \times K}$ | Repeats |
|---|---|---|---|---|---|---|
| ***SLLGRM-dimer*** *[SEQ ID NO. 5]* | 0.145 ±0.023 | 208200 ±31284 | 4.89 ±0.74 | -25316 ±2748 | -62 ±10 | 5 |

(continued)

| Peptide [SEQ ID NO. of monomer] | N | $K_a$ $\frac{1}{M}$ | $K_D$ μM | ΔH $\frac{cal}{mol}$ | ΔS $\frac{cal}{mol \times K}$ | Repeats |
|---|---|---|---|---|---|---|
| ***Pep2-dimer*** *[SEQ ID NO. 5]* | 0.157 ±0.014 | 524000 ±107000 | 1.91 ±0.39 | -29890 ±3100 | -76 ±n/a | 1 |
| ***Pep1-dimer*** *[SEQ ID NO. 10]* | 0.189 ±0.035 | 5752500 ±5798628 | 0.312 ±0.211 | -25868 ±3922 | -58 ±15 | 4 |
| ***P1dC*** *[SEQ ID NO. 15]* | 0.196 ±0.005 | 2486667 ±228384 | 0.42 ±0.038 | -21700 ±624 | -45 ±2 | 3 |

Furthermore, we have noticed that the baselines of the thermograms of the Pep2- and the Pep1-dimers showed strong fluctuations in form of many endothermic and exothermic peaks (Figure 3), suggesting protein aggregation. To evaluate the probable aggregation, we have measured the turbidity of HBc solutions with increasing concentrations of the peptidic dimers. All peptides showed increased turbidity with increasing concentrations, with Pep1- and Pep2-dimers inducing turbidity at low micromolar concentrations, while the SLLGRM-dimer induced turbidity at high micromolar concentrations, in accordance with their $K_D$ values (Figure 4). The turbidity increase of HBc solution upon the addition of the peptidic dimers further implied peptide-induced aggregation of core particles.

Motivated by the aggregating properties of the peptidic dimers and their possible interference with the virion assembly we have decided to modify the highest-affinity Pep1-based dimer to enable its in-vivo application. The peptidic dimer needs to be delivered within the cell to affect the HBV core protein. To this end we have introduced a thiol carrying moiety, cysteine, within the dimer architecture, creating a thiolated Pep1-dimer analogue, P1dC (Figure 2D and F). The thiol enables cell delivery with the help of thiol-reactive cell penetrating peptides, that are cleaved off the active molecule within the cell cytosol (Schneider, Benz, *et al.,* 2021; Schneider, Kithil, *et al.,* 2021). P1dC retained the nanomolar binding affinity to HBV core protein (Figure 5A,B, Table 1), while the control scrambled analogue scrP1dC showed no binding to the viral core protein (Figure 5C,D).

EM-maps of HBc capsids with bound SLLGRM dimers or P1dC confirmed that the peptides bind to the tips of the spikes (Figure 6). The length of the resolved peptide density accommodates some 6 amino acids for both constructs. This suggests that a similar core binds to the tips of the spikes. Interestingly, SLLGRM dimers bind to both spikes of the asymmetric unit, while monomeric SLLGRM (SEQ ID DO. 5) binds only to the spikes closest to the threefold-symmetry axis (Makbul, Khayenko, *et al.,* 2021). This implies that dimerization allows addressing the less affine binding site on HBc-capsids, which was impossible with the equivalent monomeric binder. The EM-maps confirm that the SLLGRM dimers and P1dC, both bind with a molar ratio of 0.25 to HBc capsids. The EM-maps did not resolve the linker region, which agrees with a flexible linker that has no specific binding site on the capsid surface. Our next goal was to demonstrate that P1dC affects HBV core protein within living mammalian cells. We incubated live HEK293 cells expressing HBc with P1dC and the scrambled scrP1dC together with the thiol-reactive cell penetrating peptide, then fixed the cells and visualized the HBV core proteins with the help of antibodies (Figure 7). Satisfyingly, we observed HBc aggregates in cells treated with P1dC, while cells incubated with the scrambled dimer showed HBc distribution similar to the untreated cells (Figure 8).

*- Discussion*

Based on known weak peptide binders of HBc we were able to develop high-affinity dimeric aggregator of the core protein, capable of HBc aggregation in living cells. We have based our strongest in-vivo binder, P1dC, on the strongest binder we identified using the ITC experiments, Pep1-dimer. It is worth mentioning that also other HBV core protein binding dimers we designed and synthesized showed aggregation potential in-vitro and this library of potential HBc aggregators could further be extended since there is a number of tolerable mutations in the binding sequence (Figure 9). Furthermore, medicinal chemistry approaches can be applied to develop an even stronger peptidic binder, by, e.g., introducing cyclization or non-natural amino acids to the binding sequence.

We observed the formation of strongly fluorescing patches within living cells upon administration of the P1dC (Figure 8) indicating the formation of HBc aggregates. The assembly of HB viruses within infected hepatocytes requires the formation of intact capsids surrounding the HBV genome followed by envelopment of the nucleocapsid and the export of virions. Our peptide dimers would likely interfere with the virion formation by aggregating HBc mono- or multimers, or even whole capsids.

P1dC is suitable as a new lead compound for the treatment of chronic hepatitis. Already several decades ago

it was demonstrated that Pep2 at high micromolar concentrations can reduce the production HB viruses to ca. 30 % in Hep G2, liver hepatitis model cells (Böttcher *et al.,* 1998). P1dC, having nanomolar affinity, evident aggregating properties, and established in-cell delivery can further reduce the production of HB viruses or even block it completely.

The following examples and drawings illustrate the present invention without, however, limiting the same thereto.

BRIEF DESCRIPTION OF THE DRAWINGS

**Figure 1.** *Structure of a Hepatitis B virus.*

**Figure 2.** *Structure of specific peptides of the present invention.*

A) SLLGRM-dimer.
B) GSLLGRMKGA-dimer (Pep2-dimer).
C) MHRSLLGRMKGA-dimer (Pep1-dimer).
D) MHRSLLGRMKGA-dimer-C (P1dC).
E) Preferred linker with a length of 80 Å, including a cysteine (thiol group).

**Figure 3.** *SLLGRM-, Pep2- and Pep1- dimers bindHBV core proteins in low micromolar to nanomolar affinities. Interaction analysis of HBV capsids and the SLLGRM-, Pep2- and Pep1- dimers by ITC.*

A) An exemplary titration of 150 μM HBc solution with 1500 μM solution of the SLLGRM-dimer. This ITC experiment was repeated 5 times under similar conditions (Table 1). A clear dose-response curve is derived from the heat injection profile.
B) A control titration of an empty HBc buffer solution with 1500 μM solution of SLLGRM-dimer. No dose-response is evident.
C) A titration of 50 μM HBc solution with 500 μM solution of the Pep2-dimer (Table 1). A clear dose-response curve is derived from the heat injection profile.
D) A control titration of an empty HBc buffer solution with 500 μM solution of Pep2-dimer. No dose-response is evident.
E) An exemplary titration of 40 μM HBc solution with 300 μM solution of the Pep1-dimer. This ITC experiment was repeated 4 times under similar conditions (Table 1). A clear dose-response curve is derived from the heat injection profile.
F) A control titration of an empty HBc buffer solution with 300 μM solution of Pep1-dimer. No dose-response is evident.

N: stoichiometry of the interaction, K: association constant, $\Delta H$: binding enthalpy and $\Delta S$: binding entropy. Chi^2/DoF describes the goodness of the fit.

**Figure 4.** *The peptidic dimers induce turbidity in HBc solutions. Assessment of HBc solution turbidity with peptide-dimers at 350 nm.*

A) 10μM HBc solution was titrated with different concentrations of peptide dimers. The filled and open circles and the triangles represent data points for the Pep1-, Pep2- and SLLGRM-dimers. The HBc protein buffer was used as reference baseline for the measurements. The peptide-dimers are well soluble in buffer A and do not show any signs of turbidity at 350 nm (data not shown). Pep1- and Pep2-dimers induce turbidity in low micromolar concentration.
B) 50μM HBc solution was titrated with different concentrations of SLLGRM-dimer. The SLLGRM-dimer induces turbidity in high micromolar concentrations.

**Figure 5.** *P1dC binds HBc with nanomolar affinity. Interaction analysis of HBV capsids with P1dC and the scrambled dimer scrP1dC by ITC.*

A) An exemplary titration of 25 μM HBc solution with 100 μM solution of P1dC. This ITC experiment was repeated 4 times under similar conditions (Table 1). A clear dose-response curve is derived from the heat injection profile.
B) A control titration of an empty HBc buffer solution with 100 μM solution of P1dC. No dose-response is evident.
C) A titration of 25 μM HBc solution with 100 μM solution of the scrambled dimer scrP1dC. No dose-response is evident.
D) A control titration of an empty HBc buffer solution with 100 μM solution of scrP1dC. No dose-response is evident.

**Figure 6.** *The peptide dimers bind to the tips of the spikes of the HBV core protein. HBc capsids with bound*

*SLLGRMdimer or P1dC imaged by electron cryomicroscopy.*

A) and B) show selected areas of micrographs of HBc capsids with bound SLLGRM-dimers (A) or with bound P1dC (B). One exemplary aggregate of multiple HBc capsids is indicated by an arrow in each micrograph.
C) and D) show close-ups of the asymmetric unit of HBc capsids with bound SLLGRM dimers (C) or with bound P1C dimers (D). Models of a single asymmetric unit consisting of two HBc dimers is fitted into the asymmetric unit. Both maps show a density at the tips of the spikes (arrow) that accounts for approximately six amino acids of the dimeric aggregator. The position of the symmetry axes of the icosahedral capsid is labelled with numbers in (C).

**Figure 7.** *Experimental setup for live cell experiments.*

A) HEK293 cells are transfected with a HBc coding plasmid and after 24 hours are expressing the protein.
B) The thiolated peptide dimers are pre-incubated with TNB-CPP, a thiol-reactive cell penetrating peptide, for 30 min, and then applied for 1 hour on live cells.
C) Wild-type and transfected live cells are used for the experiment.
D) After the application of the peptide dimers on transfected live cells, the cells are fixed, labelled for HBc with antibodies and imaged with a wide-field microscope.

**Figure 8.** *Transfection experiments with P1dC and scrP1dC.*

Exemplary images of wild-type and HBc expressing mammalian cells that were treated either with P1dC or the scrambled scrP1dC. The wild-type cells show no fluorescence, indicating the lack of HBc expression and the lack of unspecific binding of the HBc antibody or the secondary antibody. The untreated transfected HEK293 cells show a homogenous fluorescence in the nucleus area and some fluorescent punctae in the cytosol, indicating a homogenous distribution of HBc in the nucleus area with some of HBc located in the cytosol. Cells treated with P1dC show an anormal morphology and large fluorescent lumps, indicating aggregates of HBc protein. Cells treated with scrP1dC show an anormal morphology but no fluorescent lumps, implying the lack of HBc aggregation.

**Figure 9.** *Mutation of the M to L, I, V or F in the core binding motif "SLLGRM" does not affect the binding.*

A) A chemiluminescence intensity peptide microarray readout of point mutations of GSLLGRMKGA-derived peptides [SEQ ID NO. 10] incubated with HBV core protein.
B) A heatmap of the readout in A). White within the grey-scale indicates greatly reduced binding or the lack of it and identifies the core binding sequence, mutations in which could abolish the binding. SLLGRM [SEQ ID NO. 5] is the core binding sequence, but, according to the microarray, M to L, I, V or F substitution would not negatively affect the binding. Additionally, R in the core binding sequence may also be substituted without significant effect on the binding. The extended binding motif, i.e., amino acids before and after SLLGRM, play a secondary role in the binding of the peptide to HBc. Possibly further mutations are tolerated within the extended motif.

EXAMPLES

Several experimental tests were performed to demonstrate the present invention and are described below. They refer to:

**Example1: Solid Phase Peptide Synthesis of the peptide analogues**

The peptides were produced using standard solid phase peptide synthesis with Fmoc chemistry. Shortly, 1% divinylbenzene Wang resin, preloaded with a 9-fluorenylmethyloxycarbonyl-Cysteine(Trityl)-OH [Fmoc-Cys(Trt)-OH] (0.4 mmol/g) was swollen in dimethylformamide (DMF) for 30 min. Then, Fmoc was removed using 20% piperidine in DMF solution and the resin was washed with DMF and dichloromethane (DCM). After washes the peptide chain was elongated by adding the desired amino acid (AA, 3 eq.) with ethylcyanohydroxyiminoacetate (Oxyma, 3 eq.) and N,N'-diisopropylcarbodiimide (DIC, 3 eq.). Capping was done with N,N-diisopropylethylamine (DIEA, 50 eq.) and acetic anhydride (50 eq.) in N-methyl-2-pyrrolidone for 30 min. Coupling efficiency was monitored by measuring the absorption of the dibenzofulvene-piperidine adduct after deprotection. The peptide chain was elongated with further identical deprotection-conjugation cycles and after the completion the peptides were cleaved from the resin using a cocktail of 94% trifluoracetic acid (TFA), 3% $H_2O$, 3% Triisopropylsilane (TIPS), for 4 hours at RT. The peptides were precipitated in ice-cold ether and then purified with HPLC and analyzed by LC-MS, as described below.

Conclusion: Peptide analogues have been synthesized and purified successfully.

**Example 2: 5-(thio)-2-nitrobenzoate activation of thiol-carrying cell penetrating peptide (CPP)**

A 10-mer oligoarginine peptide connected through di-8-Amino-3,6-dioxaoctanoic acid (DOA) linker to a cysteine (C-DOA-DOA-RRRRRRRRRR; SEQ ID NO. 24) was reacted with 10 equivalents of 5,5-dithio-bis-(2-nitrobenzoic acid) in 1: 1 DMF : 0.1 M phosphate buffer for 30 min with agitation at room temperature (RT). Then the reaction mixture was directly injected in semi-preparative HPLC, purified and analyzed by LC-MS, as described below.

Conclusion: The peptide analogues have been successfully activated.

**Example 3: Purification and characterization of peptides**

The crude peptides and peptidic compounds were purified by reverse phase HPLC using water acetonitrile gradient with 0.1% formic acid (FA). LC-MS validation was performed with similar gradient and LC-MS grade solvents. Semi-preparative HPLC was performed on Shimadzu Prominence equipped with a diode-array detector (DAD) system using a C18 reverse-phase column (Phenomenex, Aschaffenburg, Germany), Onyx Monolithic HD-C18 100×4.6 mm or Onyx Monolithic C18 100×10 mm). Purity and structural identity were verified using a DAD equipped 1260 Infinity II HPLC with a C18 reverse-phase column (Onyx Monolithic C18 50×2 mm), coupled to a mass selective detector single quadruple system (Agilent Technologies, Santa Clara, CA, US) in ESI+ mode.

Conclusion: The peptides and peptide analogous have been successfully purified and characterized.

**Example 4: Automated Solid-Phase Peptide Synthesis**

μSPOT peptide arrays (Dikmans et al., 2006) were synthesized using a MultiPep RSi robot (CEM GmbH, Kamp-Lintford, Germany) on in-house produced, acid labile, amino-functionalized, cellulose membrane discs containing 9-fluorenylmethyloxycarbonyl-β-alanine (Fmoc-β-Ala) linkers (average loading: 130 nmol/disc-4 mm diameter). Synthesis was initiated by Fmoc deprotection using 20% piperidine in DMF followed by washing with DMF and ethanol (EtOH). Peptide chain elongation was achieved using a coupling solution consisting of preactivated amino acids (0.5 M) with Oxyma (1 M) and DIC (1 M) in DMF (1:1:1, aa:oxyma:DIC). Couplings were carried out for 3 × 30 min, followed by capping (4% acetic anhydride in DMF) and washes with DMF and EtOH. Synthesis was finalized by deprotection with 20% piperidine in DMF (2 × 4 μL/disc for 10 min each), followed by washing with DMF and EtOH. Dried discs were transferred to 96 deep-well blocks and treated, while shaking, with side-chain deprotection solution, consisting of 90% TFA, 2% DCM, 5% $H_2O$ and 3% TIPS (150 μL/well) for 1.5 h at RT. Afterward, the deprotection solution was removed and the discs were solubilized overnight (ON) at RT, while shaking, using a solvation mixture containing 88.5% TFA, 4% trifluoromethanesulfonic acid (TFMSA), 5% H2O and 2.5% TIPS (250 μL/well). The resulting peptide-cellulose conjugates (PCCs) were precipitated with ice-cold ether (0.7 mL/well) and spun down at 2000 × g for 10 min at 4 °C, followed by two additional washes of the formed pellet with ice-cold ether. The resulting pellets were dissolved in DMSO (250 μL/well) to give final stocks. PCC solutions were mixed 2:1 with saline-sodium citrate buffer (150 mM NaCl, 15 mM trisodium citrate, pH 7.0) and transferred to a 384-well plate. For transfer of the PCC solutions to white-coated CelluSpot blank slides (76 × 26 mm, Intavis AG Peptide Services GmbH and CO. KG, Tübingen, Germany), a SlideSpotter (CEM GmbH) was used. After completion of the printing procedure, slides were left to dry ON.

Conclusion: Peptides were successfully displayed in array format.

**Example 5: Peptide Microarray-Binding Assay**

The microarray slides were blocked for 60 min in 5% (w/v) skimmed milk powder (Carl Roth) phosphate-buffered saline (PBS; 137 mM NaCl, 2.7 mM KCl, 10 mM $Na_2HPO_4$, 1.8 mM $KH_2PO_4$, pH 7.4). After blocking, the slides were incubated for 30 min with 55 nM (monomer equivalent) of HBc in the blocking buffer, then washed 3 times with PBS. HBc was detected with a primary 1:2500 diluted mAb16988 (anti-hepatitis B virus antibody, core antigen, clone C1-5, aa 74-89, Millipore Sigma, Darmstadt, Germany) and a secondary 1:5000 diluted HRP-coupled Anti-mouse antibody (#31430, Invitrogen). The antibodies were applied in blocking buffer for 30 min, with three PBS washes between the antibodies and after applying the secondary antibody. The chemiluminescent readout was obtained using SuperSignal West Femto maximum sensitive substrate (Thermo Scientific GmbH, Schwerte, Germany) with a c400 Azure imaging system (lowest sensitivity, 10 seconds exposure time). Binding intensities were quantified with ImageJ using the "microarray profile" plugin (OptiNav Inc, Bellevue, WA, USA). The raw grayscale intensities for each position were obtained for the left and right sides of the internal duplicate on each microarray slide, n = 4 arrays in total. Blank spots were used to determine the average background grayscale value that was subtracted from the raw grayscale intensities of non-blank spots. Afterward, the spot intensities were normalized to the average grayscale value of the 15 replicates of peptide binder P2 (GSLLGRMKGA; SEQ ID NO. 10).

HBV core protein binding was successfully determined in microarray format.

Abbreviations used:

**Pep1** MHRSLLGRMKGA [SEQ ID NO. 15],
**Pep2** GSLLGRMKGA [SEQ ID NO. 10],
**Pep1 dimer** MHRSLLGRMKGA PEG linked dimer, MW 3985 Da
**Pep2 dimer** GSLLGRMKGA PEG linked dimer, MW 3250 Da
**P1dC** P1-dimer which was modified to enter the cells, MW 4088 Da
**scrPIdC** scrambled version of P1dC, MW 4088 Da

**Example 6: Protein purification HBc CLPs**

The HBV core protein forms, when over-expressed in *E. coli,* capsid like particles (CLP) of icosahedral symmetry with triangulation numbers of T=4 or T=3. For the sake of simplicity, we call theses CLPs as capsids and the HBV core protein as HBc. In this study we used exclusively capsids formed by recombinant fl wt HBc. Nevertheless, all numbers for concentrations, ITC parameters etc. refer to a monomeric HBc. We prepared the capsids according to the same procedure as described before (Makbul, Kraft, *et al.,* 2021). Our purification procedure delivers mainly (ca. 95 %) T =4 capsids with 240 HBc subunits. A small fraction has T = 3 with 180 HBc subunits (data not shown).
Conclusion: HBc CLPs were successfully purified

**Example 7: Isothermal Titration Calorimetry (ITC)**

Purified and pooled fractions (ca 8 ml) of capsids were filtered (Rotilabo syringe filter with a pore size of 220 nm, Carl Roth GmbH Co. KG, Karlsruhe, Germany), dialysed against 1.4 L buffer A (40 mM HEPES, 200 mM NaCl, 1 mM MgCl2, 1 mM CaCl2, pH 7.5) using a dialysis membrane tube (Spectra Por Biotech cellulose ester tube, 1 MDa MWCO, Spectrum Laboratories, Inc., Rancho Dominguez, CA, USA). The dialysis was performed at 4 °C under gentle stirring for 16 h overnight. Next day, the dialysed sample was removed from the dialysis tube and concentrated in a centrifuge using a concentrator (30 kDa MWCO Spin-X UF 6 mL, Corning Inc., Corning, NY, USA). Before the determination of the concentration by the Bradford assay (Roti Nanoquant, Carl Roth GmbH Co. KG, Karlsruhe, Germany) the concentrate was filtered (centrifugal filter unit Ultrafree MC, pore size of 100 nm, Merck KGaA, Darmstadt, Germany). The dimeric peptides were dissolved in the buffer obtained from the dialysis of capsids. In this buffer the SLLGRM-dimers and P2-dimers have solubilities of at least 8 mM and the P1-dimer of at least 2 mM. Before filling the ITC cell and syringe, all samples were degassed for 10 minutes at 20 °C (ThermoVac, Malvern Panalytical, Malvern, Worcestershire, UK). Using a MicroCal iTC200 instrument (Malvern Panalytical, Malvern, Worcestershire, UK) solutions of dimeric peptides were titrated into solutions of capsids according to specifications in the Table 2. The resulting thermograms and isotherms were processed and fitted by the Origin software supplied with the iTC200 instrument. The thermograms were integrated and the corresponding isotherms were fitted using a one site model. All obtained thermodynamic parameters refer to concentrations of monomeric HBc. All ITC experiments were complemented with control experiments where solutions of dimeric peptides were titrated into dialysis buffer.
Conclusion: Direct binding of the dimeric peptides was confirmed in solution. Binding stoichiometry, binding affinity and thermodynamic parameters were determined.

***Table 2*** iTC200 instrument's specifications used for the interaction analysis between peptide-dimers and capsids.

| | | | |
|---|---|---|---|
| Number of injections | 14-16 | Injection parameters: | |
| Cell Temperature (° C) | 20 | Volume (μl) | 2.5 - 2.8 |
| Reference power (μCal/s) | 11 | duration (s) | 5 - 5.6 |
| Initial delay (s) | 60 -180 | Spacing (s) | 180/240 |
| Syringe concentration (mM) | 0.1 - 0.75 | Filter period (s) | 5/1 |
| Cell concentration (mM) | 0.025 - 0.15 fl wt HBc | Cell volume (ml) | 0,2009 |
| Stirring speed (rpm) | 600 | | |
| Feedback mode | high | | |
| First injection | No first injection | | |

**Example 8: Turbidity assay**

All peptides were dissolved in buffer A and the capsid solutions were filtered once (centrifugal filter unit Ultrafree MC, pore size of 100 nm, Merck KGaA, Darmstadt, Germany). The concentrations of the P1-, P2- and SLLGRM-dimers were varied between 0.1-100 μM and the concentration of HBc kept constant at 10 μM for the P1- and P2-dimer and at 50 μM for the SLLGRM-dimer. All experiments were performed using a standard photometer (GENESYS™ UV/VIS spectral photometer, Thermo Fisher Scientific, Hillsboro, OR, USA) at RT and at a wavelength of 350 nm using disposable UV transparent cuvettes.

Conclusion: The propensity to induce aggregation of HBVags in vitro was successfully determined for the tested dimers.

**Example 9: Cryogenic grid preparation of capsids in complex with peptide-dimers**

In a plasma cleaner (model PDC-002. Harrick Plasma, Ithaca, NY, USA) holey carbon grids (R1.2/1.3, 300 mesh Cu grids, Quantifoil Micro Tools, Jena, Germany) were made hydrophilic by plasma discharging. This was done at a pressure of 29 Pa for 2 minutes using ambient air as plasma medium at "medium power" of the instrument. First, solutions of purified HBc (200 μM) in complex with the P1dC- and the SLLGRM-dimer (each 400 μM) were prepared in buffer A and 3.5 μl aliquots of each sample were applied onto the grids. For plunge freezing of grids ethane was used as medium (liquefied by liquid nitrogen) with the help of a Vitrobot (mark IV, FEI Company, Hillsboro, OR, USA) using filter papers of Whatman (type 541). The Vitrobot had following settings: no wait and drain times, 6 s of blot time, blot force of 25 and a nominal humidity of 100 %. The frozen grids were stored in liquid nitrogen for at least one night before being used for image acquisition.

Conclusion: Capsids and Capsids complexed with the dimers were successfully prepared for Cryo-EM imaging.

**Example 10: Cryo-EM and image processing**

Cryo-EM was done as previously described (Makbul, Kraft, et al., 2021). In brief: Movies were acquired with the software EPU on a Krios G3 electron microscope equipped with a Falcon III camera (Thermo Fisher Scientific, Hillsboro, OR, USA) in linear mode at a magnification of 75,000 with an accelerating voltage of 300 kV. The total exposure was 40 e-/$Å^2$ and was fractionated across 20 fractions. For HBc capsids with bound P1C dimers, 3 movies were acquired per hole and one hole was acquired per stage position. For HBc capsids with bound SLLGRM dimers, at each stage position three movies were acquired per hole from the central hole and the four closest neighbouring holes. The different movie positions at the same stage position were centered with image shift. Each image shift position was treated as a different optics group in the subsequent image processing. Image processing was done with Relion 3.1 as previously described (Makbul, Kraft, et al., 2021) imposing icosahedral symmetry. At the end of the image processing with relion, the particles images of the final map were imported to CryoSparc 4.02 and were further refined with none uniform (Punjani et al., 2020), including global and local CTF refinement and Ewald's sphere correction. The final 3D-maps from the CryoSparc none unidform refinement were filtered with deepemhancer for better interpretability.

Conclusion: Cryo-EM and image processing for Capsids complexed with the dimers was successfully conducted.

**Example 11: Modelling of Cryo-EM maps, refinement of PDB files and their validation**

For modelling of the EM densities of HBc in complex with the dimeric peptides the PDB file 7od6 (Makbul, Khayenko, *et al.,* 2021) was used as starting model. This model represents the asymmetric unit of the HBc capsids with T =4. After slight modifications, the PDB model was fitted into the EM-map as a rigid body and refined iteratively by the software packages Coot (Casañal *et al.,* 2020) and Phenix (Liebschner *et al.,* 2019) and validated by Molprobidity (Prisant *et al.,* 2020). The EM-map used for model building corresponds to a section of the entire EM-map generated by Chimera. The resolution of the density at the tips of the capsids which we attributed to the binding segments of the dimeric peptides was low. Therefore, these densities could only be modelled as poly-alanine chains. All figures showing EM-densities with or without the corresponding PDB models were prepared with Chimera (Yang *et al.,* 2012). Conclusion: Cryo-EM maps were successfully refined and validated for Capsids complexed with the dimers.

**Example 12: Cloning**

Full length wild type (fl wt) HBc (genotype D; strain ayw; GenBank: V01460.1, MQLFHLCLIISCSCPTVQASKLCLGWLWGMDIDPYKEFGATVELSFLPSDFFPSVRDL LDTASALYREALESPEHCSPHHTALRQAILCWGELMTLATWVGVNLEDPASRDLVVS

YVNTNMGLKFRQLLWFHISCLTFGRETVIEYLVSFGVWIRTPPAYRPPNAPILSTLPET TVVRRRGRSPRRRTPSPRRRRSQSPRRRRSQSRESQC; SEQ ID NO. 25) (Galibert et al., 1979) was cloned into the pEGFP-C2 vector (Clontech) using Gibson assembly (Gibson, 2011) by replacing the gene sequence coding for eGFP. The vector and insert were amplified by PCR and purified by gel extraction (FastGene Gel/PCR Extraction Kit, Nippon Genetics Europe GmbH, Düren, Germany). The purified PCR products were assembled into a single plasmid construct using a home-made Gibson assembly reaction mixture. An aliquot of the reaction product was transformed into XL1 blue cells, plated onto LB-amp agar-plates and grown at 37 °C ON. Six colonies were used for the inoculation of 6 × 5 ml LB-amp medium. The cell cultures were grown under vigorous shaking in an incubator at 37 °C ON. Next day, the plasmid DNA was extracted from the cell cultures (FastGene Plasmid Mini Kit, Nippon Genetics Europe GmbH, Düren, Germany) and sequenced by Sanger sequencing (Microsynth Seqlab GmbH, Göttingen, Germany). A plasmid construct containing the correct gene sequence of HBc was used for endotoxin-free plasmid DNA preparation (NucleoBond Xtra Midi EF, MACHEREY-NAGEL GmbH & Co. KG, Düren, Germany).

Conclusion: Full length wild type HBc (genotype D; strain ayw; GenBank: V01460.1) was successfully cloned into the pEGFP-C2 vector.

**Example 13: HEK293 cell cultures and transfection**

HEK293 cells were cultured in DMEM (GIBCO), supplemented with GlutaMax and pyruvate (GIBCO), 10% fetal bovine serum (GIBCO) and 1% Penicillin/Streptomycin (Sigma) at 37°C and with 5% $CO_2$. The cells were plated on 0.15 mm thick 18 mm glass coverslips coated with 35 μg/ml Poly-D-Lysine in a 12-well plate and were transfected with the cloned 1 μg plasmid DNA per coverslip using PEI (Polyethylenimine). The transfection was performed at 60-80% confluence. Shortly before transfection the medium was changed to fresh DMEM. The DNA was added to 100 μl DMEM without additives and mixed, 4 μl fresh PEI (1 mg/ml) was added, mixed immediately and incubated for 20 min at RT. The transfection mix was pipetted dropwise on cells while swirling, and incubated overnight. The medium was changed to fresh DMEM with 2% FBS after 12-24 hours, and on the following day the cells were used for live assays, then fixed and stained.

Conclusion: HEK293 cells were successfully cultured and transfected with HBc.

**Example 14: Cell assays**

Live HEK293 cells expressing HBc were incubated with 10 μM P1dC and with 10 μM of the scrambled version of the peptide, both peptides in-situ activated with the reactive CPP. After the incubation the treated and the untreated live HEK293 cells expressing HBc and also wild-type HEK293 cells were washed and fixed with 0.1 M sodium phosphate buffer pH 7.4 containing 4% paraformaldehyde (EM grade, Polysciences) and 1% sucrose for 10-20 min at 37°C. After three rinses in phosphate buffered saline (PBS), the cells were permeabilized with 0.1% Triton X-100 in PBS for 10 min at RT, rinsed again and blocked for 1 h in PBS with 3% bovine serum albumin (BSA). Then, primary mAb16988 (MilliporeSigma) and secondary DyLight650 (#84545, Invitrogen) antibodies were applied sequentially with 1:500 dilution in blocking solution for 1 hour.Wide field fluorescence microscopy

The coverslips with the cell samples were inserted in an imaging chamber (Ludin Chamber Type 1, Life Imaging Services) and imaged in PBS. The measurements were taken from distinct samples with a sample size ≥ 2, for each group. A series of images, used to generate the datapoints, were acquired from different regions of the sample, each region having a distinct group of cells.

The samples were imaged on an inverted Leica DMI6000B microscope with a 100x oil-immersion objective (NA 1.49) using a Leica DFC9000 GTC VSC-05760 sCMOS camera (16-bit, image pixel size: 130 nm). The 628/40 excitation and 692/40 emission filter was used for DyLight650, 10 images were acquired at a frame rate (exposure time) of 100 ms and constant illumination intensity to ensure comparability. n≥10.

Conclusion: Aggregation of HBc was confirmed for P1dC. In transfected HEK293 cells.

The features disclosed in the foregoing description, in the claims and/or in the accompanying drawings may, both separately and in any combination thereof, be material for realizing the invention in diverse forms thereof.

REFERENCES

Block, T. M., Chang, K.-M., & Guo, J.-T. (2021). Prospects for the Global Elimination of Hepatitis B. Annual Review of Virology, 8(1), 437-458. https://doi.org/10.1146/annurev-virology-091919-062728

Böttcher, B., Tsuji, N., Takahashi, H., Dyson, M. R., Zhao, S., Crowther, R. A., & Murray, K. (1998). Peptides that block hepatitis B virus assembly: analysis by cryomicroscopy, mutagenesis and transfection. The EMBO Journal,

17(23), 6839-6845. https://doi.org/10.1093/emboj/17.23.6839

Casañal, A., Lohkamp, B., & Emsley, P. (2020). Current developments in Coot for macromolecular model building of Electron Cryo-microscopy and Crystallographic Data. Protein Science, 29(4), 1055-1064. https://doi.org/10.1002/pro.3791

Cox, A. L., El-Sayed, M. H., Kao, J. H., Lazarus, J. v., Lemoine, M., Lok, A. S., & Zoulim, F. (2020). Progress towards elimination goals for viral hepatitis. Nature Reviews Gastroenterology and Hepatology, 17(9), 533-542. https://doi.org/10.1038/s41575-020-0332-6

Dikmans, A., Beutling, U., Schmeisser, E., Thiele, S., & Frank, R. (2006). SC2: A novel process for manufacturing multipurpose high-density chemical microarrays. QSAR and Combinatorial Science, 25(11), 1069-1080. https://doi.org/10.1002/qsar.200640130

Dyson, M. R., & Murray, K. (1995). Selection of peptide inhibitors of interactions involved in complex protein assemblies: association of the core and surface antigens of hepatitis B virus. Proceedings of the National Academy of Sciences, 92(6), 2194-2198. https://doi.org/10.1073/pnas.92.6.2194

Galibert, F., Mandart, E., Fitoussi, F., Tiollais, P., & Chamay, P. (1979). Nucleotide sequence of the hepatitis B virus genome (subtype ayw) cloned in E. coli. Nature, 281(5733), 646-650. https://doi.org/10.1038/281646a0

Gibson, D. G. (2011). Enzymatic Assembly of Overlapping DNA Fragments (pp. 349-361). https://doi.org/10.1016/B978-0-12-385120-8.00015-2

Gripon, P., Cannie, I., & Urban, S. (2005). Efficient Inhibition of Hepatitis B Virus Infection by Acylated Peptides Derived from the Large Viral Surface Protein. Journal of Virology, 79(3), 1613-1622. https://doi.org/10.1128/JVI.79.3.1613-1622.2005

Hu, J., Protzer, U., & Siddiqui, A. (2019). Revisiting Hepatitis B Virus: Challenges of Curative Therapies. Journal of Virology, 93(20). https://doi.org/10.1128/JVI.01032-19

Liebschner, D., Afonine, P. v., Baker, M. L., Bunkóczi, G., Chen, V. B., Croll, T. I., Hintze, B., Hung, L.-W., Jain, S., McCoy, A. J., Moriarty, N. W., Oeffner, R. D., Poon, B. K., Prisant, M. G., Read, R. J., Richardson, J. S., Richardson, D. C., Sammito, M. D., Sobolev, O. v., ... Adams, P. D. (2019). Macromolecular structure determination using X-rays, neutrons and electrons: recent developments in Phenix. Acta Crystallographica Section D Structural Biology, 75(10), 861-877. https://doi.org/10.1107/S2059798319011471

Makbul, C., Khayenko, V., Maric, H. M., & Böttcher, B. (2021). Conformational Plasticity of Hepatitis B Core Protein Spikes Promotes Peptide Binding Independent of the Secretion Phenotype. Microorganisms, 9(5), 956. https://doi.org/10.3390/microorganisms9050956

Makbul, C., Kraft, C., Crrießmann, M., Rasmussen, T., Katzenberger, K., Lappe, M., Pfarr, P., Stoffer, C., Stöhr, M., Wandinger, A.-M., & Böttcher, B. (2021). Binding of a Pocket Factor to Hepatitis B Virus Capsids Changes the Rotamer Conformation of Phenylalanine 97. Viruses, 13(11), 2115. https://doi.org/10.3390/v13112115

Prisant, M. G., Williams, C. J., Chen, V. B., Richardson, J. S., & Richardson, D. C. (2020). New tools in MolProbity validation: CaBLAM for CryoEM backbone, UnDowser to rethink "waters," and NGL Viewer to recapture online 3D graphics. Protein Science, 29(1), 315-329. https://doi.org/10.1002/pro.3786

Punjani, A., Zhang, H., & Fleet, D. J. (2020). Non-uniform refinement: adaptive regularization improves single-particle cryo-EM reconstruction. Nature Methods, 17(12), 1214-1221. https://doi.org/10.1038/s41592-020-00990-8

Schneider, A. F. L., Benz, L. S., Lehmann, M., & Hackenberger, C. P. R. (2021). Cell-Permeable Nanobodies Allow Dual-Color Super-Resolution Microscopy in Untransfected Living Cells. Angewandte Chemie International Edition, 60(40), 22075-22080. https://doi.org/10.1002/anie.202103068

Schneider, A. F. L., Kithil, M., Cardoso, M. C., Lehmann, M., & Hackenberger, C. P. R. (2021). Cellular uptake of large biomolecules enabled by cell-surface-reactive cell-penetrating peptide additives. Nature Chemistry, 13(6), 530-539. https://doi. org/10.103 8/s415 57-021-00661-x

Tian, J., Li, C., & Li, W. (2021). Entry of hepatitis B virus: going beyond NTCP to the nucleus. Current Opinion in Virology, 50, 97-102. https://doi.org/10.1016/j.coviro.2021.08.001

Volz, T., Allweiss, L., MBarek, M. ben, Warlich, M., Lohse, A. W., Pollok, J. M., Alexandrov, A., Urban, S., Petersen, J., Lütgehetmann, M., & Dandri, M. (2013). The entry inhibitor Myrcludex-B efficiently blocks intrahepatic virus spreading in humanized mice previously infected with hepatitis B virus. *Journal of Hepatology, 58*(5), 861-867. https://doi.org/10.1016/j.jhep.2012.12.008

Watashi, K., & Wakita, T. (2015). Hepatitis B Virus and Hepatitis D Virus Entry, Species Specificity, and Tissue Tropism. Cold Spring Harbor Perspectives in Medicine, 5(8), a021378. https://doi.org/10.1101/cshperspect.a021378

World Health Organisation. (2022). Hepatitis B fact sheet. https://www.who.int/en/news-room/fact-sheets/detail/hepatitis-b

Yang, Z., Lasker, K., Schneidman-Duhovny, D., Webb, B., Huang, C. C., Pettersen, E. F., Goddard, T. D., Meng, E. C., Sali, A., & Ferrin, T. E. (2012). UCSF Chimera, MODELLER, and IMP: An integrated modeling system. Journal of Structural Biology, 179(3), 269-278. https://doi.org/10.1016/j.jsb.2011.09.006

# SEQUENCE LISTING

| | | |
|---|---|---|
| **1** | **Sequence Listing Information** | |
| 1-1 | File Name | U31150EP_sequence listing_2.xml |
| 1-2 | DTD Version | V1_3 |
| 1-3 | Software Name | WIPO Sequence |
| 1-4 | Software Version | 2.1.0 |
| 1-5 | Production Date | 2023-03-02 |
| 1-6 | Original free text language code | en |
| 1-7 | Non English free text language code | |
| **2** | **General Information** | |
| 2-1 | Current application: IP Office | EP |
| 2-2 | Current application: Application number | |
| 2-3 | Current application: Filing date | |
| 2-4 | Current application: Applicant file reference | U31150EP |
| 2-5 | Earliest priority application: IP Office | |
| 2-6 | Earliest priority application: Application number | |
| 2-7 | Earliest priority application: Filing date | |
| 2-8de | Applicant name | Julius-Maximilians-Universität Würzburg |
| 2-8en | Applicant name: Name Latin | Julius-Maximilians-Universitaet Wuerzburg |
| 2-9 | Inventor name | |
| 2-9en | Inventor name: Name Latin | |

| | | |
|---|---|---|
| 2-10en | Invention title | HBV core protein binding compounds and uses thereof |
| 2-11 | Sequence Total Quantity | 25 |
| **3-1** | **Sequences** | |
| 3-1-1 | Sequence Number [ID] | 1 |
| 3-1-2 | Molecule Type | AA |
| 3-1-3 | Length | 6 |
| 3-1-5 | Residues | XXLGXX |
| **3-2** | **Sequences** | |
| 3-2-1 | Sequence Number [ID] | 2 |
| 3-2-2 | Molecule Type | AA |
| 3-2-3 | Length | 6 |
| 3-2-4-1 | Features Location/Qualifiers | **source** 1..6<br>mol_type= protein<br>organism= synthetic construct |
| | NonEnglishQualifier Value | |
| 3-2-4-2 | Features Location/Qualifiers | **VARIANT** 6<br>note= X is selected from methionine (M), valine (V), leucine (L), isoleucine (I), phenylalanine (F), and non-natural amino acids comprising hydrophobic exchange(s) |
| | NonEnglishQualifier Value | |
| 3-2-5 | Residues | SLLGRX 6 |
| **3-3** | **Sequences** | |
| 3-3-1 | Sequence Number [ID] | 3 |
| 3-3-2 | Molecule Type | AA |
| 3-3-3 | Length | 10 |
| 3-3-4-1 | Features Location/Qualifiers | **source** 1..10<br>mol_type= protein<br>organism= synthetic construct |
| | NonEnglishQualifier Value | |
| 3-3-4-2 | Features Location/Qualifiers | **PEPTIDE** 7 |
| | NonEnglishQualifier Value | |
| 3-3-4-3 | Features Location/Qualifiers | **VARIANT** 7<br>note= X is selected from methionine (M), valine (V), leucine (L), isoleucine (I), phenylalanine (F), and non-natural amino acids comprising hydrophobic exchange(s) |
| | NonEnglishQualifier Value | |
| 3-3-5 | Residues | GSLLGRXKGA 10 |
| **3-4** | **Sequences** | |
| 3-4-1 | Sequence Number [ID] | 4 |
| 3-4-2 | Molecule Type | AA |
| 3-4-3 | Length | 12 |

| | | |
|---|---|---|
| 3-4-4-1 | Features Location/Qualifiers | **source** 1..12<br>mol_type= protein<br>organism= synthetic construct |
| | NonEnglishQualifier Value | |
| 3-4-4-2 | Features Location/Qualifiers | **VARIANT** 11<br>note= X is selected from methionine (M), valine (V), leucine (L), isoleucine (I), phenylalanine (F), and non-natural amino acids comprising hydrophobic exchange(s) |
| | NonEnglishQualifier Value | |
| 3-4-5 | Residues | MHRSLLGRXK GA 12 |
| **3-5** | **Sequences** | |
| 3-5-1 | Sequence Number [ID] | 5 |
| 3-5-2 | Molecule Type | AA |
| 3-5-3 | Length | 6 |
| 3-5-4-1 | Features Location/Qualifiers | **source** 1..6<br>mol_type= protein<br>organism= synthetic construct |
| | NonEnglishQualifier Value | |
| 3-5-5 | Residues | SLLGRM 6 |
| **3-6** | **Sequences** | |
| 3-6-1 | Sequence Number [ID] | 6 |
| 3-6-2 | Molecule Type | AA |
| 3-6-3 | Length | 6 |
| 3-6-4-1 | Features Location/Qualifiers | **source** 1..6<br>mol_type= protein<br>organism= synthetic construct |
| | NonEnglishQualifier Value | |
| 3-6-5 | Residues | SLLGRL 6 |
| **3-7** | **Sequences** | |
| 3-7-1 | Sequence Number [ID] | 7 |
| 3-7-2 | Molecule Type | AA |
| 3-7-3 | Length | 6 |
| 3-7-4-1 | Features Location/Qualifiers | **source** 1..6<br>mol_type= protein<br>organism= synthetic construct |
| | NonEnglishQualifier Value | |
| 3-7-5 | Residues | SLLGRI 6 |
| **3-8** | **Sequences** | |
| 3-8-1 | Sequence Number [ID] | 8 |
| 3-8-2 | Molecule Type | AA |
| 3-8-3 | Length | 6 |

| | | |
|---|---|---|
| 3-8-4-1 | Features Location/Qualifiers | **source** 1..6<br>mol_type= protein<br>organism= synthetic construct |
| | NonEnglishQualifier Value | |
| 3-8-5 | Residues | SLLGRV 6 |
| **3-9** | **Sequences** | |
| 3-9-1 | Sequence Number [ID] | 9 |
| 3-9-2 | Molecule Type | AA |
| 3-9-3 | Length | 6 |
| 3-9-4-1 | Features Location/Qualifiers | **source** 1..6<br>mol_type= protein<br>organism= synthetic construct |
| | NonEnglishQualifier Value | |
| 3-9-5 | Residues | SLLGRF 6 |
| **3-10** | **Sequences** | |
| 3-10-1 | Sequence Number [ID] | 10 |
| 3-10-2 | Molecule Type | AA |
| 3-10-3 | Length | 10 |
| 3-10-5 | Residues | GSLLGRMKGA 10 |
| **3-11** | **Sequences** | |
| 3-11-1 | Sequence Number [ID] | 11 |
| 3-11-2 | Molecule Type | AA |
| 3-11-3 | Length | 10 |
| 3-11-4-1 | Features Location/Qualifiers | **source** 1..10<br>mol_type= protein<br>organism= synthetic construct |
| | NonEnglishQualifier Value | |
| 3-11-5 | Residues | GSLLGRLKGA 10 |
| **3-12** | **Sequences** | |
| 3-12-1 | Sequence Number [ID] | 12 |
| 3-12-2 | Molecule Type | AA |
| 3-12-3 | Length | 10 |
| 3-12-4-1 | Features Location/Qualifiers | **source** 1..10<br>mol_type= protein<br>organism= synthetic construct |
| | NonEnglishQualifier Value | |
| 3-12-5 | Residues | GSLLGRIKGA 10 |
| **3-13** | **Sequences** | |
| 3-13-1 | Sequence Number [ID] | 13 |
| 3-13-2 | Molecule Type | AA |

| | | |
|---|---|---|
| 3-13-3 | Length | 10 |
| 3-13-4-1 | Features Location/Qualifiers | **source** 1..10<br>mol_type= protein<br>organism= synthetic construct |
| | NonEnglishQualifier Value | |
| 3-13-5 | Residues | GSLLGRVKGA 10 |
| **3-14** | **Sequences** | |
| 3-14-1 | Sequence Number [ID] | 14 |
| 3-14-2 | Molecule Type | AA |
| 3-14-3 | Length | 10 |
| 3-14-4-1 | Features Location/Qualifiers | **source** 1..10<br>mol_type= protein<br>organism= synthetic construct |
| | NonEnglishQualifier Value | |
| 3-14-5 | Residues | GSLLGRFKGA 10 |
| **3-15** | **Sequences** | |
| 3-15-1 | Sequence Number [ID] | 15 |
| 3-15-2 | Molecule Type | AA |
| 3-15-3 | Length | 12 |
| 3-15-4-1 | Features Location/Qualifiers | **source** 1..12<br>mol_type= protein<br>organism= synthetic construct |
| | NonEnglishQualifier Value | |
| 3-15-5 | Residues | MHRSLLGRMK GA 12 |
| **3-16** | **Sequences** | |
| 3-16-1 | Sequence Number [ID] | 16 |
| 3-16-2 | Molecule Type | AA |
| 3-16-3 | Length | 12 |
| 3-16-4-1 | Features Location/Qualifiers | **source** 1..12<br>mol_type= protein<br>organism= synthetic construct |
| | NonEnglishQualifier Value | |
| 3-16-5 | Residues | MHRSLLGRLK GA 12 |
| **3-17** | **Sequences** | |
| 3-17-1 | Sequence Number [ID] | 17 |
| 3-17-2 | Molecule Type | AA |
| 3-17-3 | Length | 12 |
| 3-17-4-1 | Features Location/Qualifiers | **source** 1..12<br>mol_type= protein<br>organism= synthetic construct |
| | NonEnglishQualifier Value | |
| 3-17-5 | Residues | MHRSLLGRIK GA 12 |

| 3-18 | Sequences | |
|---|---|---|
| 3-18-1 | Sequence Number [ID] | 18 |
| 3-18-2 | Molecule Type | AA |
| 3-18-3 | Length | 12 |
| 3-18-4-1 | Features Location/Qualifiers | **source** 1..12<br>mol_type= protein<br>organism= synthetic construct |
| | NonEnglishQualifier Value | |
| 3-18-5 | Residues | MHRSLLGRVK GA 12 |
| **3-19** | **Sequences** | |
| 3-19-1 | Sequence Number [ID] | 19 |
| 3-19-2 | Molecule Type | AA |
| 3-19-3 | Length | 12 |
| 3-19-4-1 | Features Location/Qualifiers | **source** 1..12<br>mol_type= protein<br>organism= synthetic construct |
| | NonEnglishQualifier Value | |
| 3-19-5 | Residues | MHRSLLGRFK GA 12 |
| **3-20** | **Sequences** | |
| 3-20-1 | Sequence Number [ID] | 20 |
| 3-20-2 | Molecule Type | AA |
| 3-20-3 | Length | 6 |
| 3-20-4-1 | Features Location/Qualifiers | **source** 1..6<br>mol_type= protein<br>organism= synthetic construct |
| | NonEnglishQualifier Value | |
| 3-20-5 | Residues | LLGRMK 6 |
| **3-21** | **Sequences** | |
| 3-21-1 | Sequence Number [ID] | 21 |
| 3-21-2 | Molecule Type | AA |
| 3-21-3 | Length | 8 |
| 3-21-4-1 | Features Location/Qualifiers | **source** 1..8<br>mol_type= protein<br>organism= synthetic construct |
| | NonEnglishQualifier Value | |
| 3-21-5 | Residues | ALLGRMKG 8 |
| **3-22** | **Sequences** | |
| 3-22-1 | Sequence Number [ID] | 22 |
| 3-22-2 | Molecule Type | AA |
| 3-22-3 | Length | 9 |

| | | |
|---|---|---|
| 3-22-4-1 | Features Location/Qualifiers | **source** 1..9<br>mol_type= protein<br>organism= synthetic construct |
| | NonEnglishQualifier Value | |
| 3-22-5 | Residues | SLLGRMKGA 9 |
| **3-23** | **Sequences** | |
| 3-23-1 | Sequence Number [ID] | 23 |
| 3-23-2 | Molecule Type | AA |
| 3-23-3 | Length | 8 |
| 3-23-4-1 | Features Location/Qualifiers | **source** 1..8<br>mol_type= protein<br>organism= synthetic construct |
| | NonEnglishQualifier Value | |
| 3-23-5 | Residues | SLLGRMKG 8 |
| **3-24** | **Sequences** | |
| 3-24-1 | Sequence Number [ID] | 24 |
| 3-24-2 | Molecule Type | AA |
| 3-24-3 | Length | 10 |
| 3-24-4-1 | Features Location/Qualifiers | **source** 1..10<br>mol_type= protein<br>organism= synthetic construct |
| | NonEnglishQualifier Value | |
| 3-24-5 | Residues | RRRRRRRRRR 10 |
| **3-25** | **Sequences** | |
| 3-25-1 | Sequence Number [ID] | 25 |
| 3-25-2 | Molecule Type | AA |
| 3-25-3 | Length | 211 |
| 3-25-4-1 | Features Location/Qualifiers | **source** 1..211<br>mol_type= protein<br>organism= Hepatitis B virus |
| | NonEnglishQualifier Value | |
| 3-25-5 | Residues | MQLFHLCLII SCSCPTVQAS KLCLGWLWGM DIDPYKEFGA TVELSFLPSD FFPSVRDLLD 60 TASALYREAL ESPEHCSPHH TALRQAILCW GELMTLATWV GVNLEDPASR DLVVSYVNTN 120 MGLKFRQLLW FHISCLTFGR ETVIEYLVSF GVWIRTPPAY RPPNAPILST LPETTVVRRR 180 GRSPRRRTPS PRRRRSQSPR RRRSQSRESQ C 211 |

## Claims

1. A compound comprising

(i) a multimer of a peptide or peptide analogue **P,**
wherein the peptide or peptide analogue **P** comprises the amino acid sequence

$X_1X_2LGX_3X_4$ SEQ ID NO. 1,

wherein

$X_1$ is selected from serine (S), glutamine (Q), asparagine (N), glycine (G), glutamate (E), aspartate (D), alanine (A), and non-natural amino acids comprising hydrophilic exchange(s),
$X_2$ is selected from leucine (L), isoleucine (I), and non-natural amino acids comprising hydrophobic exchange(s),
$X_3$ is selected from arginine (R), phenylalanine (F), glutamic acid (E) and non-natural amino acids comprising hydrophobic or hydrophilic exchange(s),
$X_4$ is selected from methionine (M), valine (V), leucine (L), isoleucine (I), phenylalanine (F), and non-natural amino acids comprising hydrophobic exchange(s),
and wherein the peptide has a length of 6 to 13 amino acids, preferably 6 to 12 amino acids,
and

(ii) a linker **L** connecting the peptides or peptide analogues **P,**
wherein the linker has a length of 60 to 160 Å, preferably 60 to 100 Å.

**2.** The compound of claim 1, wherein

$X_1$ is serine (S), and/or
$X_2$ is leucine (L), and/or
$X_3$ is arginine (R), and/or
$X_4$ is selected from methionine (M), valine (V), leucine (L), isoleucine (I) and phenylalanine (F), preferably L or M, wherein, preferably, the peptide or peptide analogue **P** comprises the amino acid sequence of

| | |
|---|---|
| SLLGR$X_4$ | SEQ ID NO. 2, |
| GSLLGR$X_4$KGA | SEQ ID NO. 3, or |
| MHRSLLGR$X_4$KGA | SEQ ID NO. 4 |

**3.** The compound of claim 1, wherein the peptide or peptide analogue **P** comprises or consists of a amino acid sequence which is selected from

| | |
|---|---|
| SLLGRM | SEQ ID NO. 5, |
| SLLGRL | SEQ ID NO. 6, |
| SLLGRI | SEQ ID NO. 7, |
| SLLGRV | SEQ ID NO. 8, |
| SLLGRF | SEQ ID NO. 9, |
| GSLLGRMKGA | SEQ ID NO. 10, |
| GSLLGRLKGA | SEQ ID NO. 11, |
| GSLLGRIKGA | SEQ ID NO. 12, |
| GSLLGRVKGA | SEQ ID NO. 13, |
| GSLLGRFKGA | SEQ ID NO. 14, |
| MHRSLLGRMKGA | SEQ ID NO. 15, |
| MHRSLLGRLKGA | SEQ ID NO. 16, |
| MHRSLLGRIKGA | SEQ ID NO. 17, |
| MHRSLLGRVKGA | SEQ ID NO. 18, and |
| MHRSLLGRFKGA | SEQ ID NO. 19. |

**4.** The compound of any one of claims 1 to 3, wherein the compound has formula I

$$(P)_m - L - (Z)_n \quad (I),$$

wherein

**P** is the peptide or peptide analogue,
**m** is at least 2, preferably, 2, 3, 4 or 5,
**L** is the linker connecting the peptides or peptide analogues **P,**
**Z** is a further moiety and **n** is 0 or 1.

**5.** The compound of claim 4, wherein m is 2 and each peptide or peptide analogue P has the same amino acid sequence and the same length (homodimer); or the two peptides or peptide analogues have a different amino acid sequence and/or a different length (heterodimer).

**6.** The compound of any one of the preceding claims, wherein the linker **L** comprises or consists of an alkane chain, diaminoacetic acid, maleimide, ethylene glycol, polyethylene glycol (PEG), *N*PEG, glycine, polyglycine, or any combination thereof.

**7.** The compound of any one of the preceding claims, wherein the linker **L** comprises at least two ethylene glycol moieties, such as 12, and at least two glycine,

and/or polyglycine,
and/or wherein the linker **L** comprises the following structure

$$-[-NH-(CH_2)_2-O-(CH_2)_2-O-(CO)-]_4-NH-(CH_2)_4-CH(R^1)-NH-[-(CO)-CH_2-O-(CH_2)_2-O-(CH_2)_2-NH]_4-,$$

and/or wherein the linker has a length of about 80 Å.

**8.** The compound of any one of claims 4 to 7, wherein the further moiety **Z** is

(a) a reactive group or a moiety comprising said reactive group, wherein said reactive group is for binding to a cell-penetrating peptide (CPP),
or
(b) a CPP,
wherein said reactive group is preferably a thiol group and said CPP is preferably a thiol-reactive CPP,
and/or wherein said compound comprising said reactive group, preferably thiol group, is a cysteine, homocysteine, 2-amino-5-mercaptopentanoic acid, alpha-methyl-cysteine, penicillamine, or 5-mercapto-norvaline.

**9.** A pharmaceutical composition comprising

a compound of any one of claims 1 to 8, and
optionally, pharmaceutically acceptable excipient(s) and/or carrier.

**10.** Use of a compound of any one of claims 1 to 8 for binding and/or aggregating the core protein of Hepatitis B virus (HBV).

**11.** The compound of any one of claims 1 to 8 or the pharmaceutical composition of claim 10 for use in medicine.

**12.** The compound of any one of claims 1 to 8 or the pharmaceutical composition of claim 10 for use in the treatment of a Hepatitis B virus (HBV) infection or a liver disease caused by HBV,
wherein the HBV infection is preferably a chronic HBV infection, and/or wherein the liver disease caused by HBV is preferably hepatitis.

**13.** The compound or pharmaceutical composition for use according to claim 12, comprising the use of a CPP, preferably a thiol-reactive CPP.

**14.** A kit comprising

(a) a compound of any one of claims 1 to 8 or a pharmaceutical composition of claim 10, and
(b) a CPP, preferably a thiol-reactive CPP.

**15.** A method for the treatment of a Hepatitis B virus (HBV) infection or a liver disease caused by HBV, comprising the administration of a therapeutically effective amount of a compound of any one of claims 1 to 8 or the pharmaceutical composition of claim 9 to a subject in need thereof,

wherein the HBV infection is preferably a chronic HBV infection, and/or wherein the liver disease caused by HBV is preferably hepatitis,
optionally comprising the co-administration of a cell penetrating peptide (CPP), preferably a thiol-reactive CPP,
wherein the administration of the compound or the pharmaceutical composition is preferably intravenous, subcutaneous, intranasal, buccal, sublingual, oral, rectal or intraperitoneal.

**Figure 1**

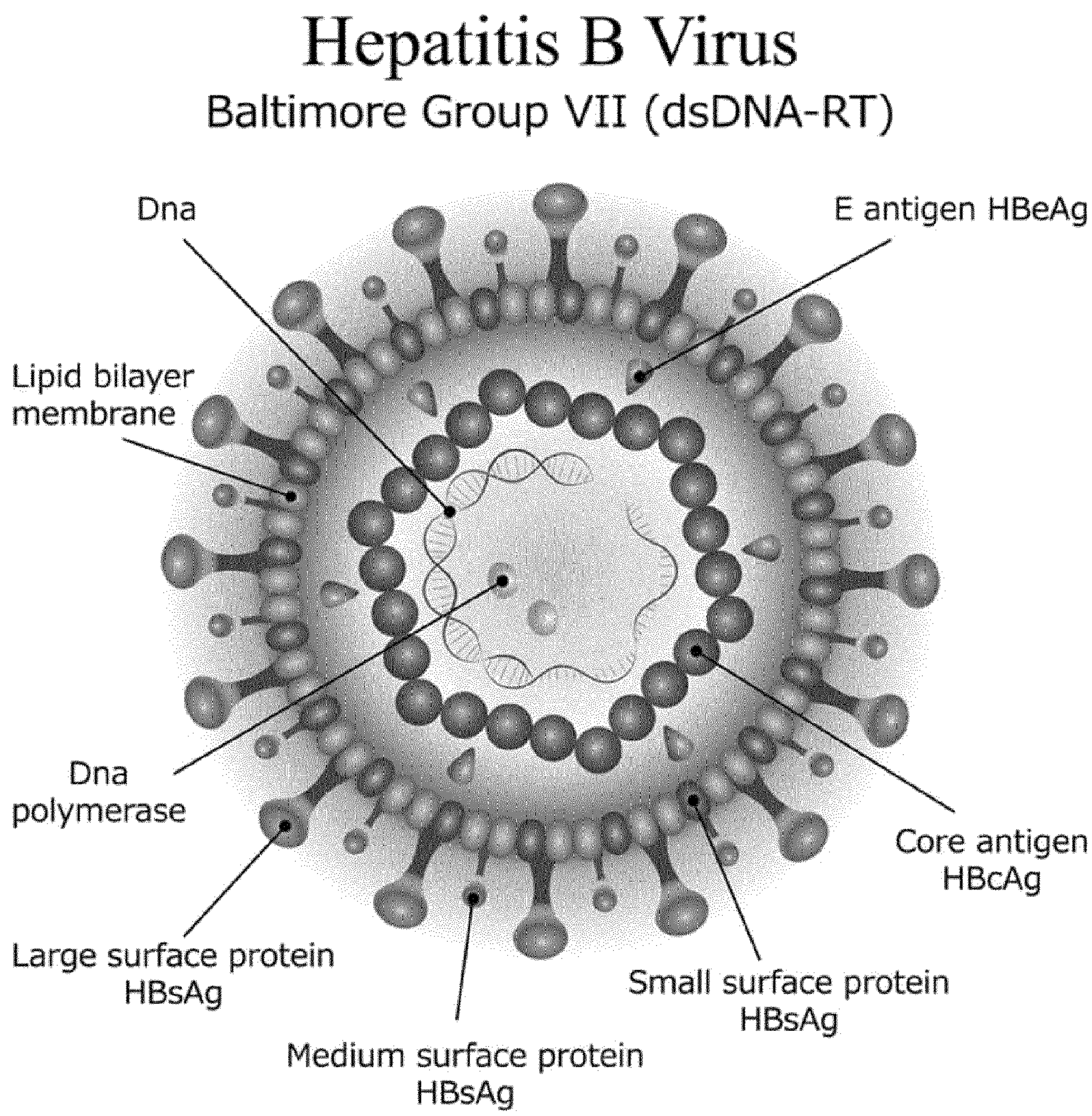
Hepatitis B Virus
Baltimore Group VII (dsDNA-RT)
Dna
E antigen HBeAg
Lipid bilayer membrane
Dna polymerase
Core antigen HBcAg
Large surface protein HBsAg
Small surface protein HBsAg
Medium surface protein HBsAg

Figure 2 A

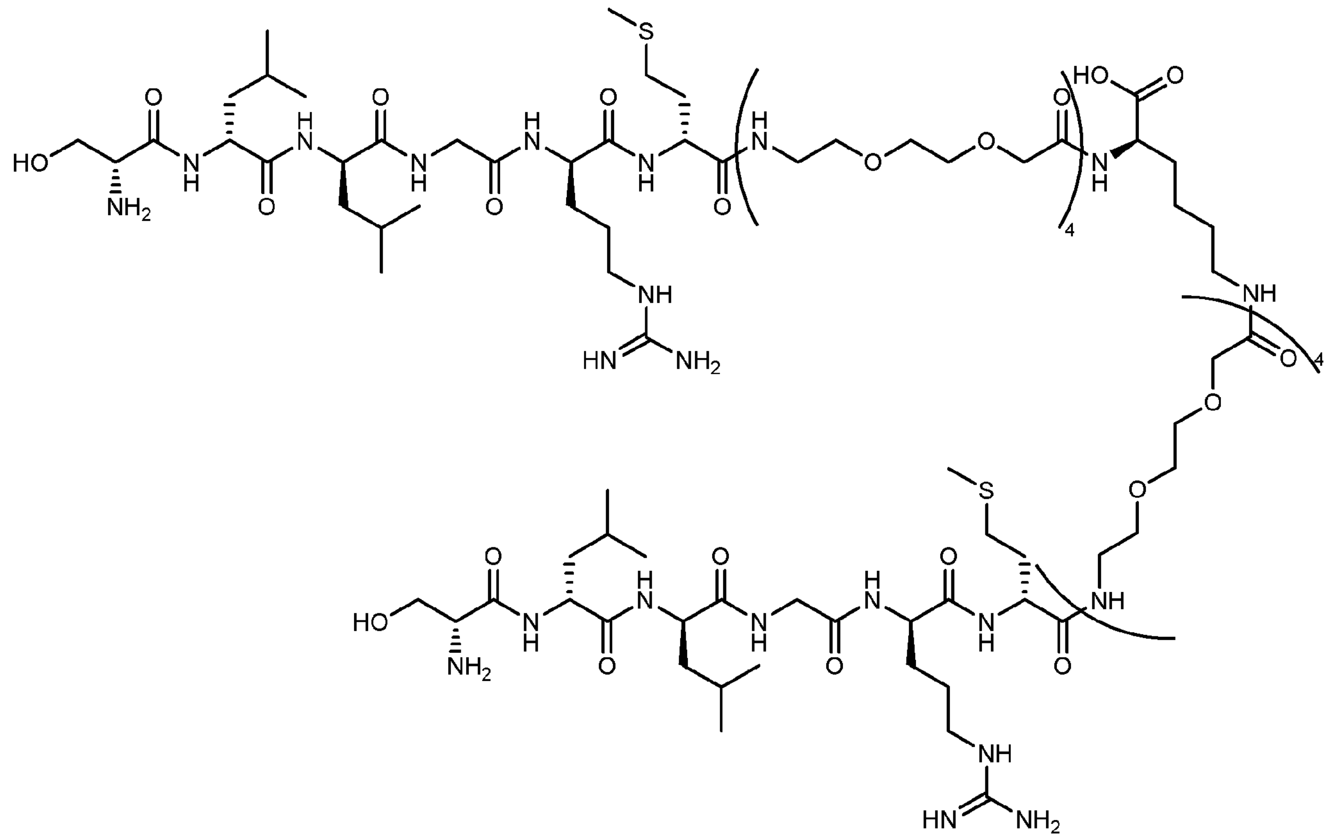

SLLGRM - dimer

P: SEQ ID NO. 5

# Figure 2 B

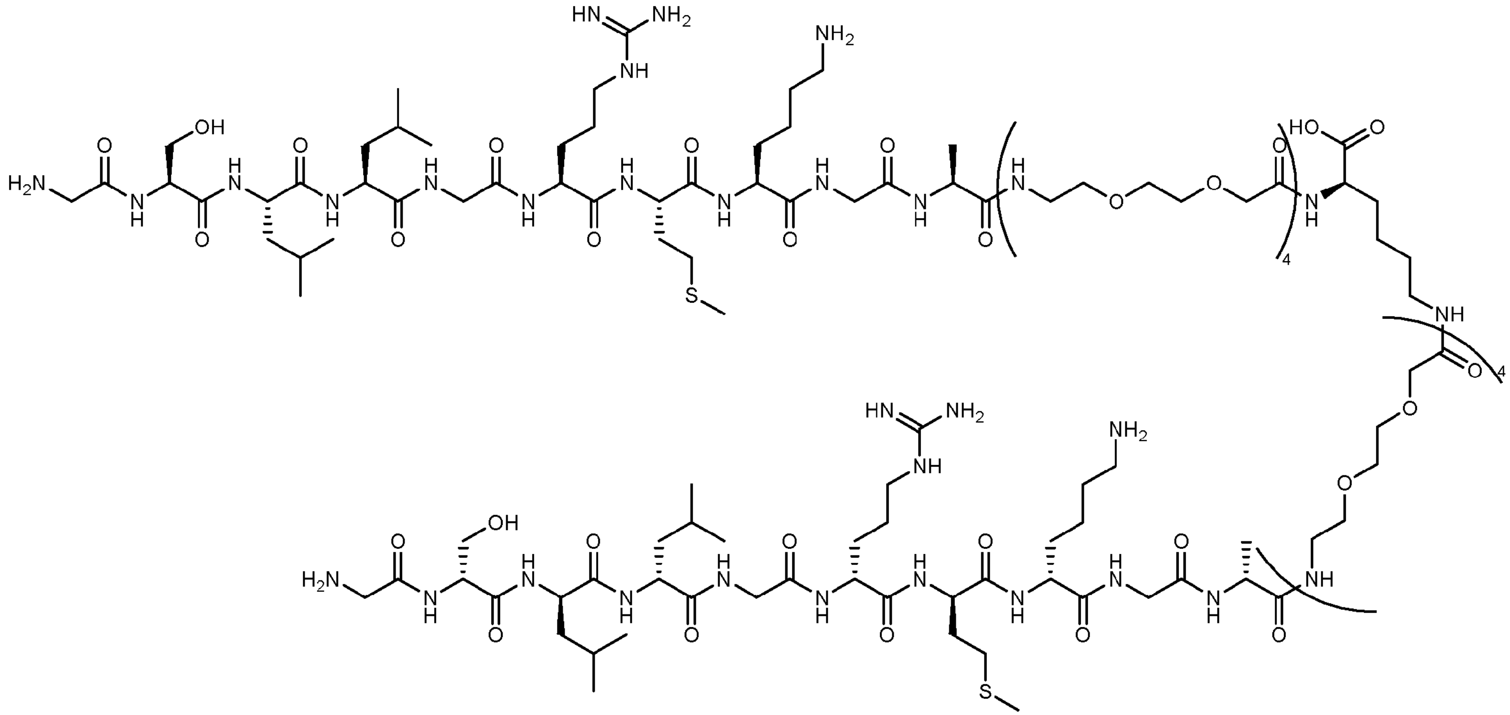

GSLLGRMKGA (Pep2) - dimer

P: SEQ ID NO. 10

# Figure 2 C

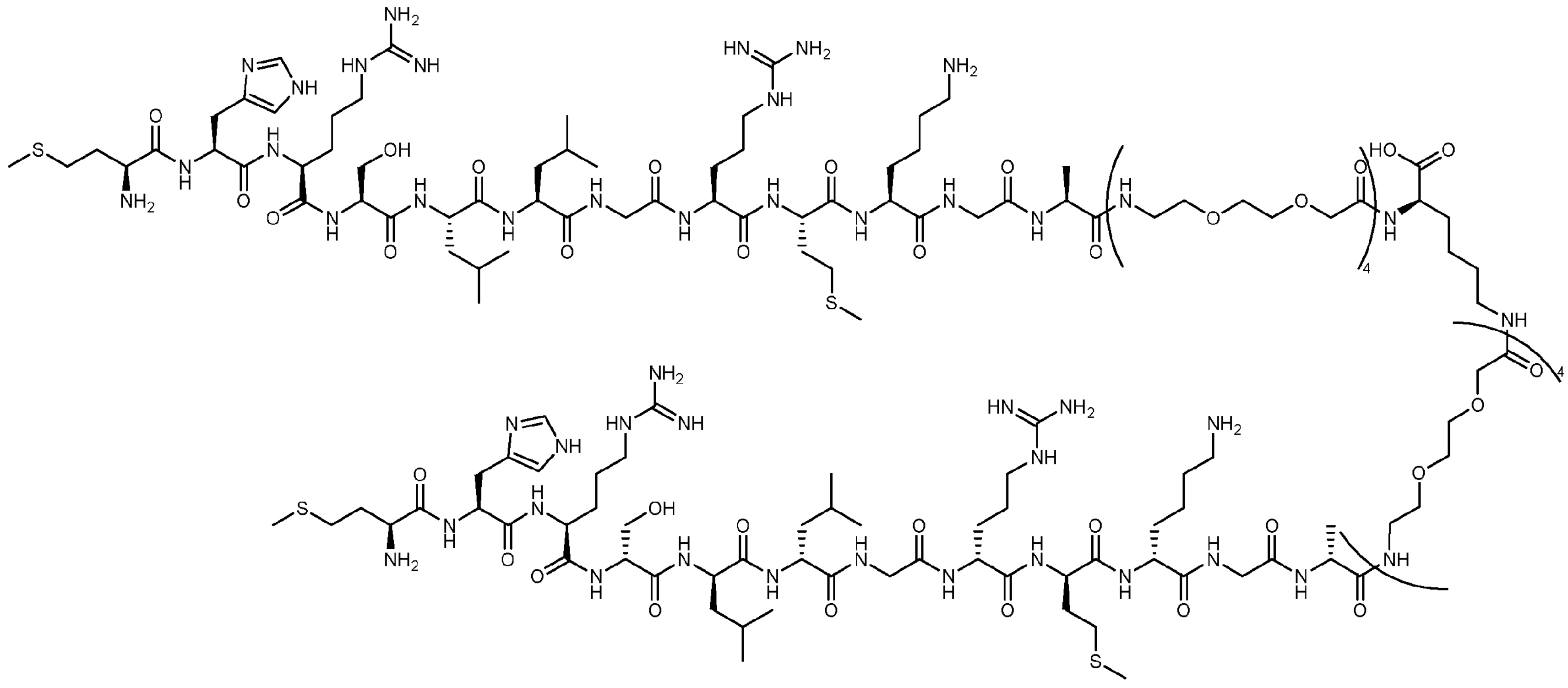

MHRSLLGRMKGA (Pep1) dimer

P: SEQ ID NO. 15

# Figure 2 D

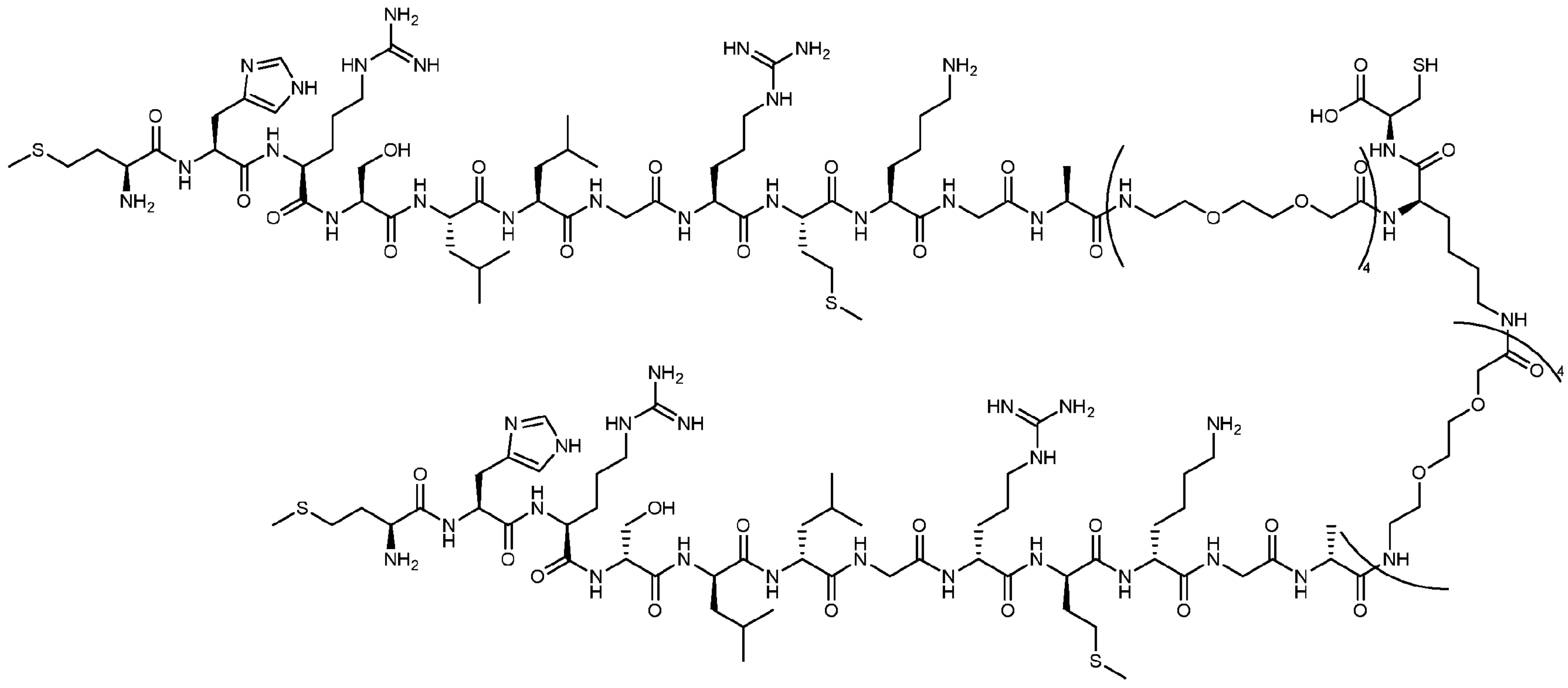

MHRSLLGRMKGA-dimer-C (P1dC)

P: SEQ ID NO. 15

Figure 2 E

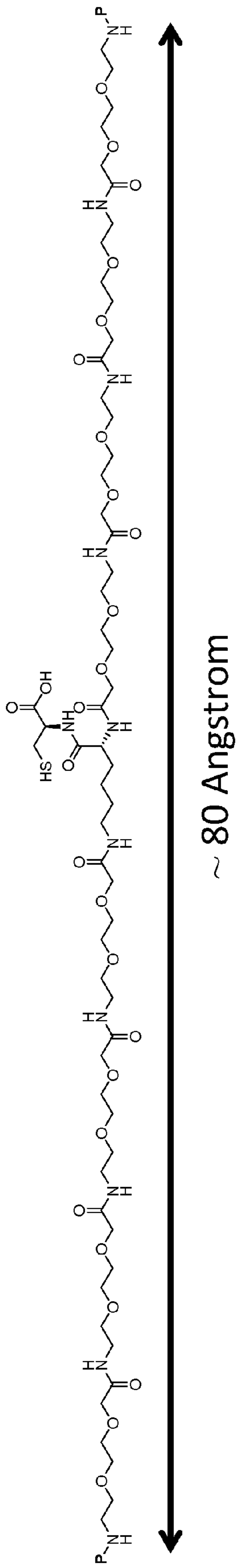

# Figure 3 A and B

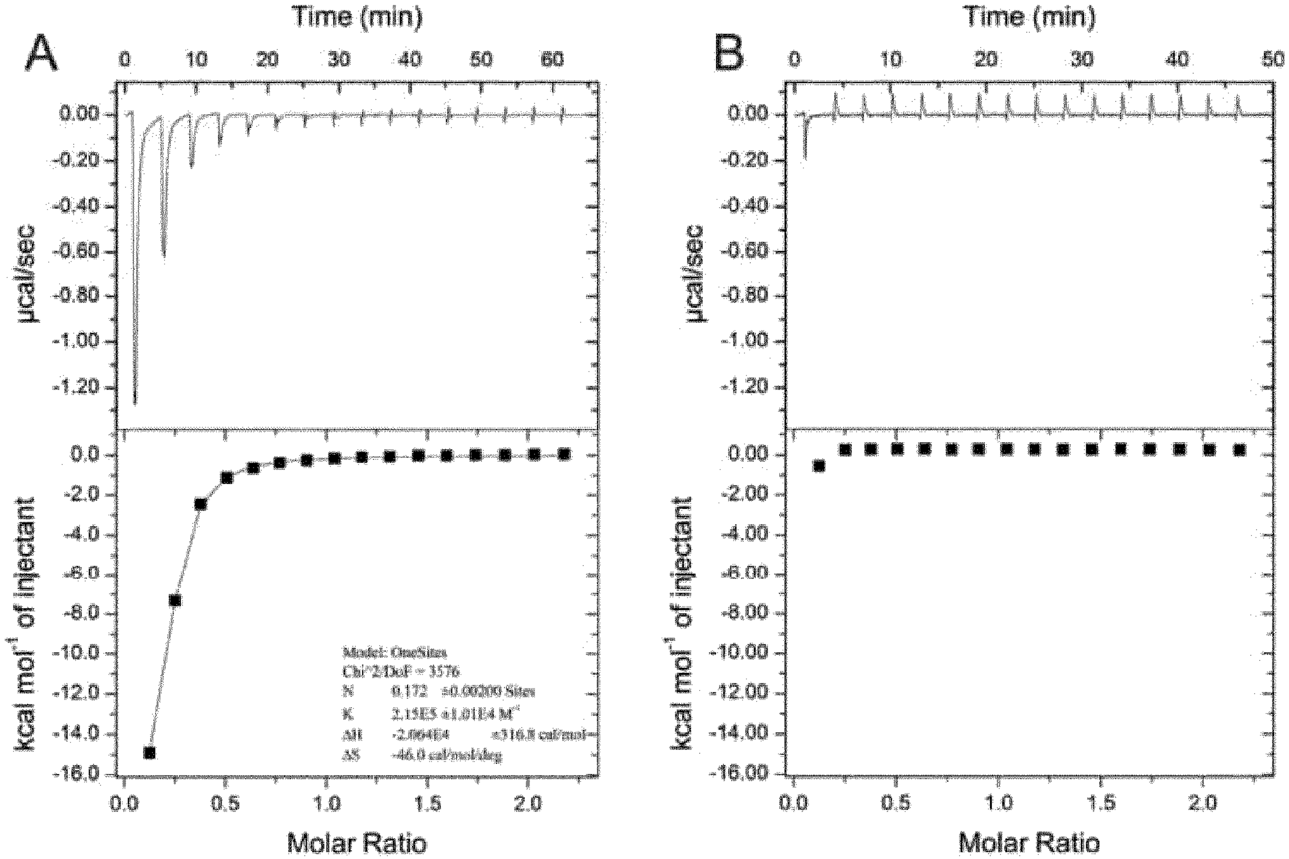


150 µM HBc
1500 µM SLLGRM-dimer

0 µM HBc
1500 µM SLLGRM-dimer

**Figure 3 C and D**

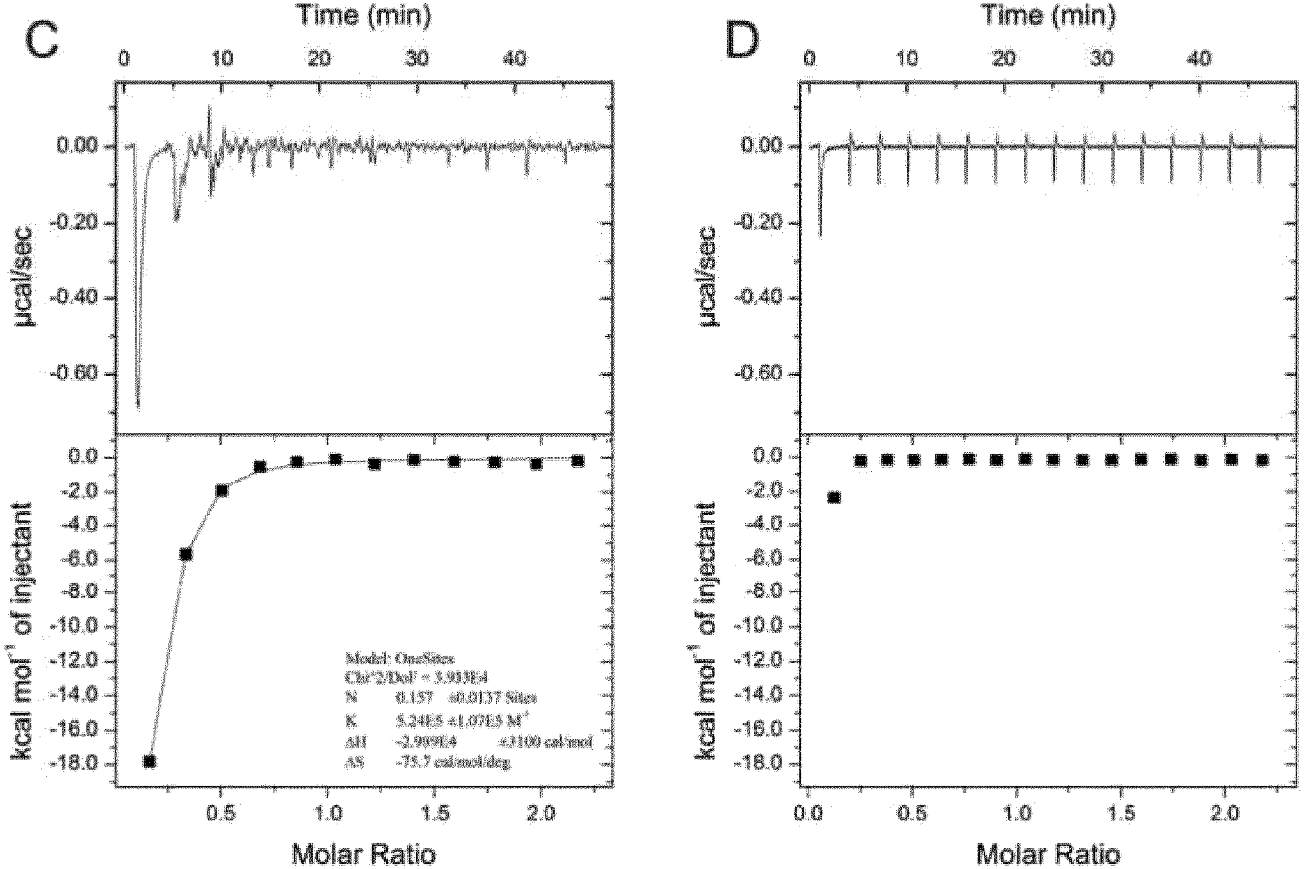


50 µM HBc
500 µM Pep2-dimer

0 µM HBc
500 µM Pep2-dimer

# Figure 3 E and F

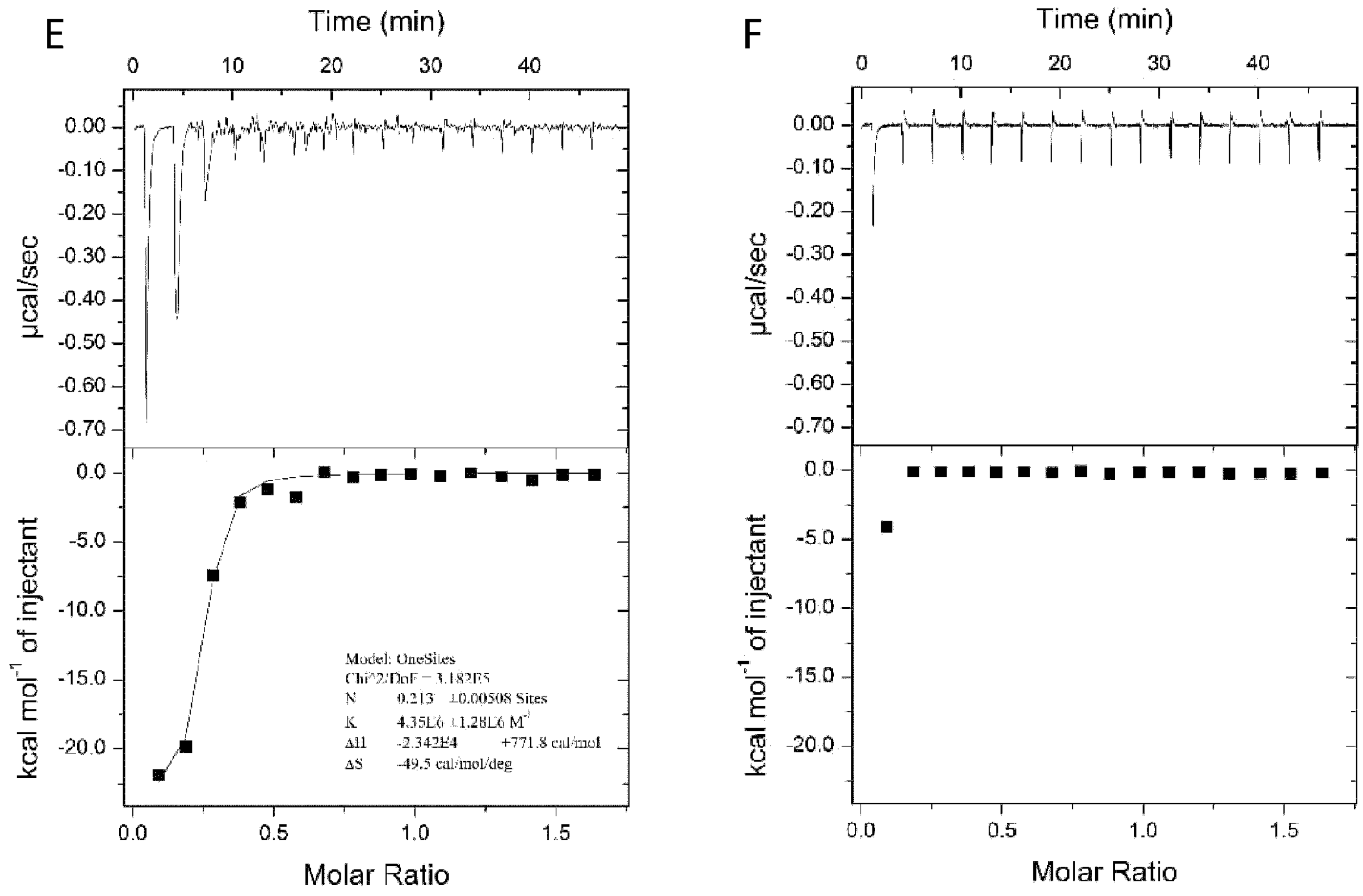


40 µM HBc
300 µM Pep1-dimer

0 µM HBc
300 µM Pep1-dimer

Figure 4

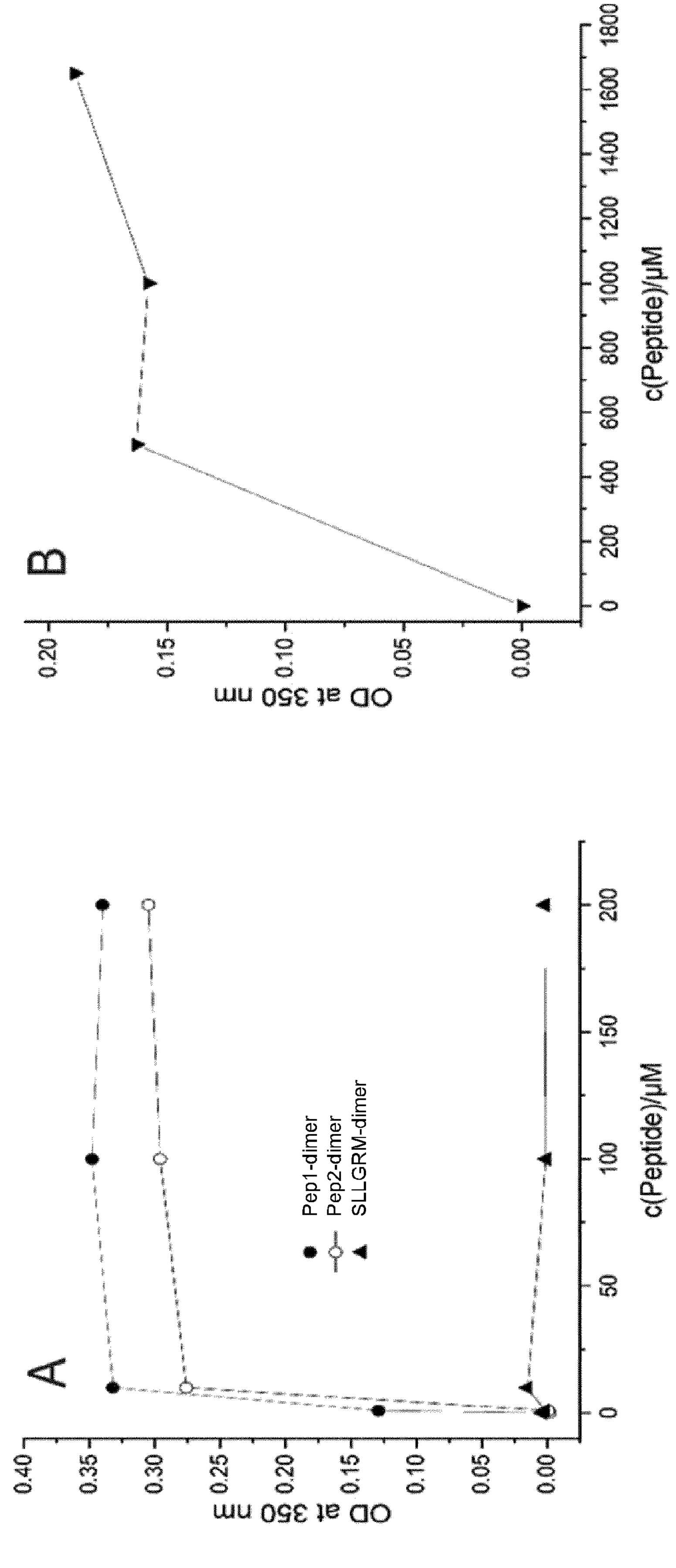
A
B
OD at 350 nm
c(Peptide)/µM
Pep1-dimer
Pep2-dimer
SLLGRM-dimer
0.00
0.05
0.10
0.15
0.20
0.25
0.30
0.35
0.40
0
50
100
150
200
400
600
800
1000
1200
1400
1600
1800

# Figure 5 A and B

A

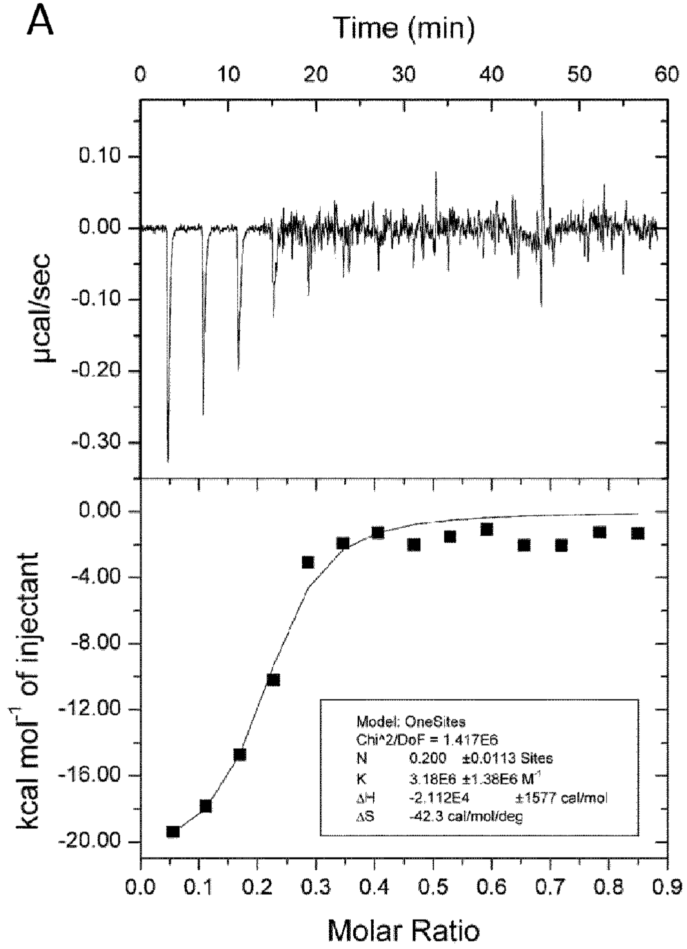


25 µM HBc
100 µM P1dC

B

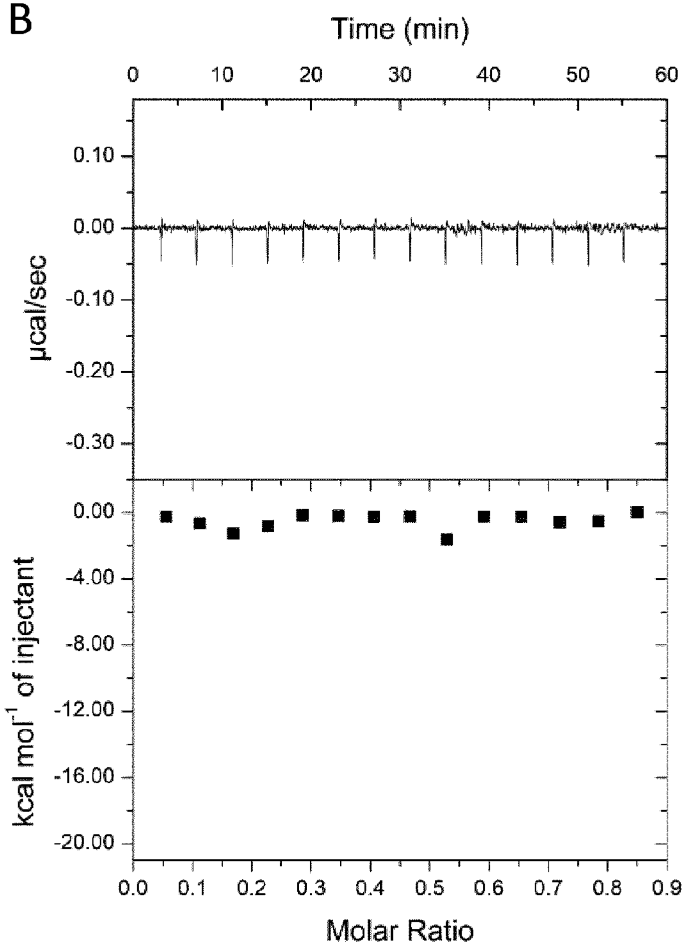


0 µM HBc
100 µM P1dC

# Figure 5 C and D

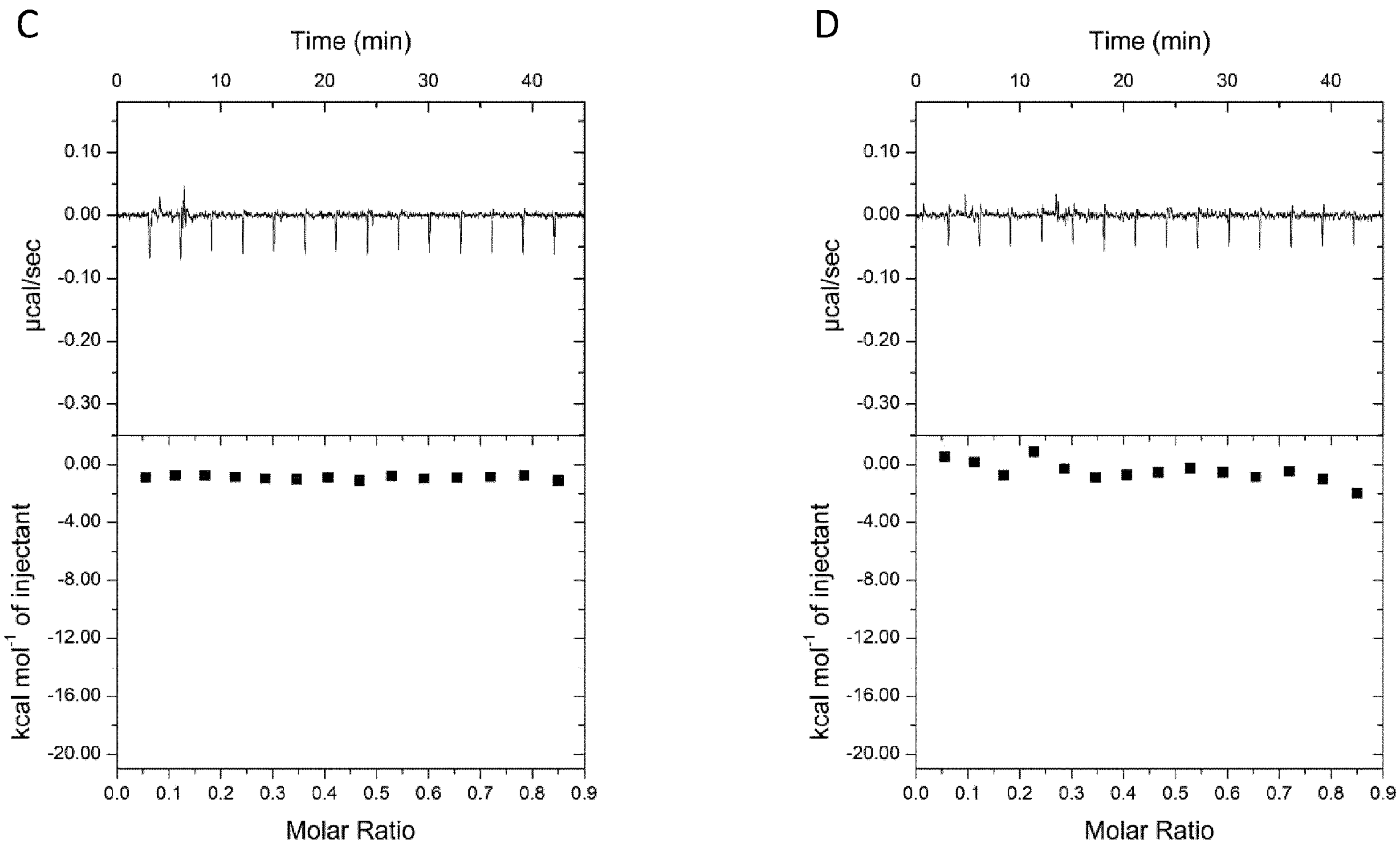


25 µM HBc
100 µM scrP1dC

0 µM HBc
100 µM scrP1dC

Figure 6

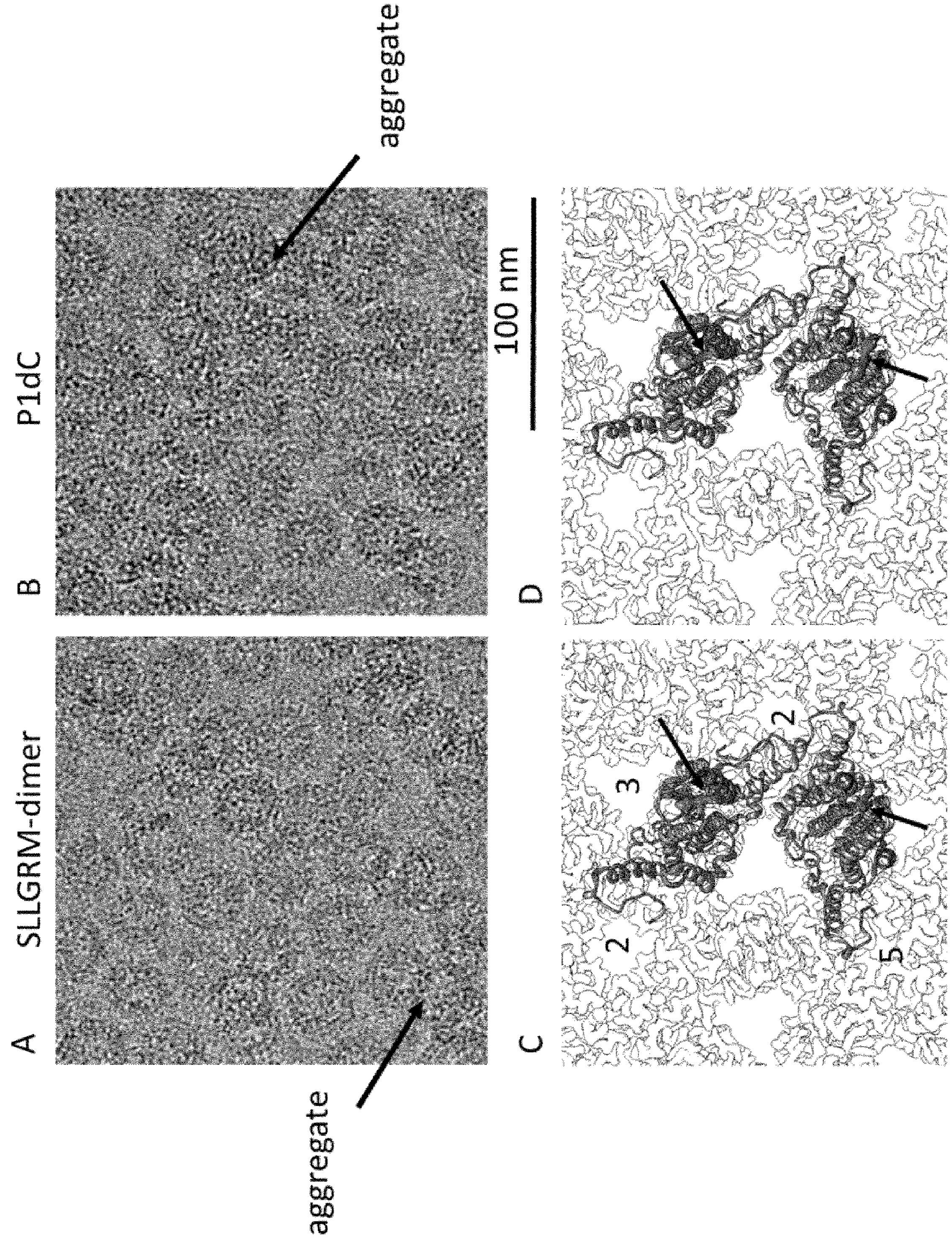
A
SLLGRM-dimer
B
P1dC
aggregate
aggregate
100 nm
C
D
2
3
2
5
10 nm

# Figure 7

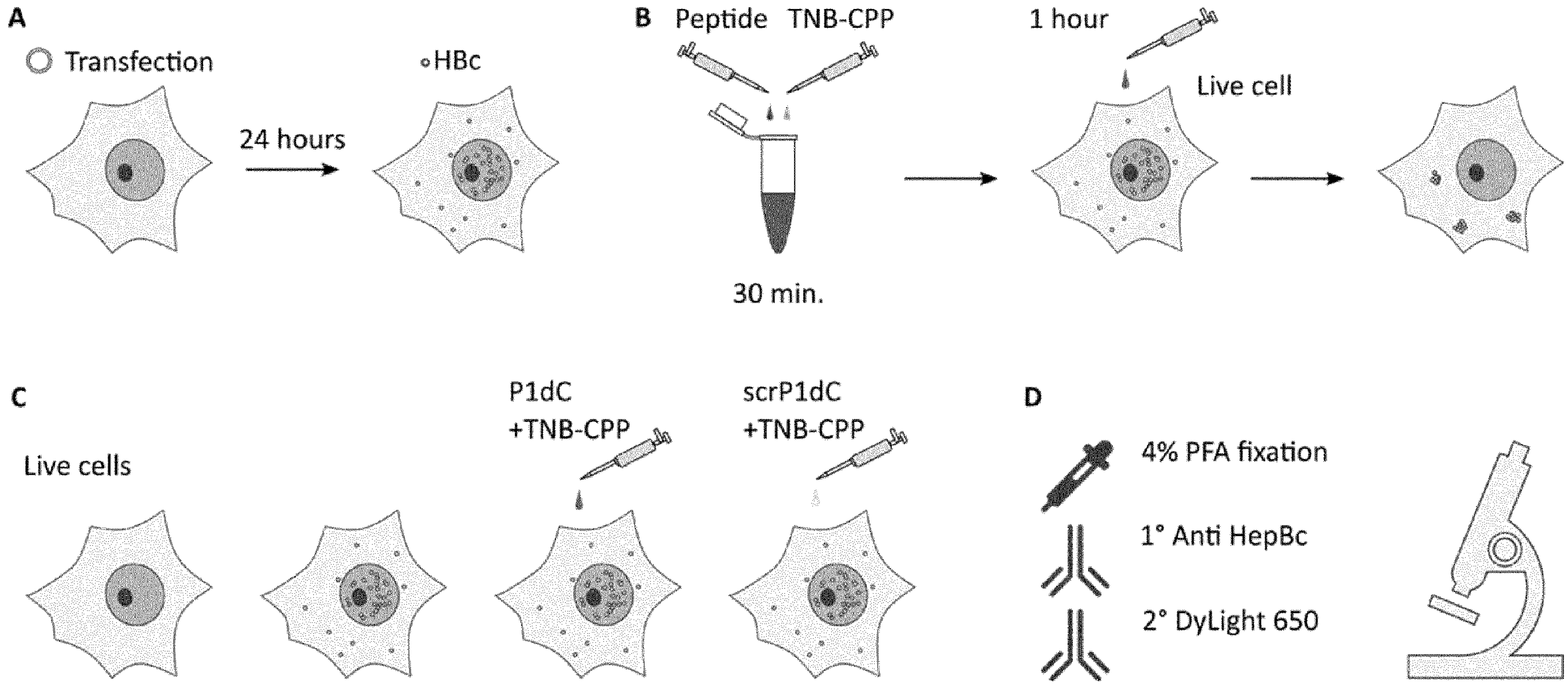
A
Transfection
24 hours
HBc
B
Peptide
TNB-CPP
30 min.
1 hour
Live cell
C
Live cells
P1dC
+TNB-CPP
scrP1dC
+TNB-CPP
D
4% PFA fixation
1° Anti HepBc
2° DyLight 650

Figure 8

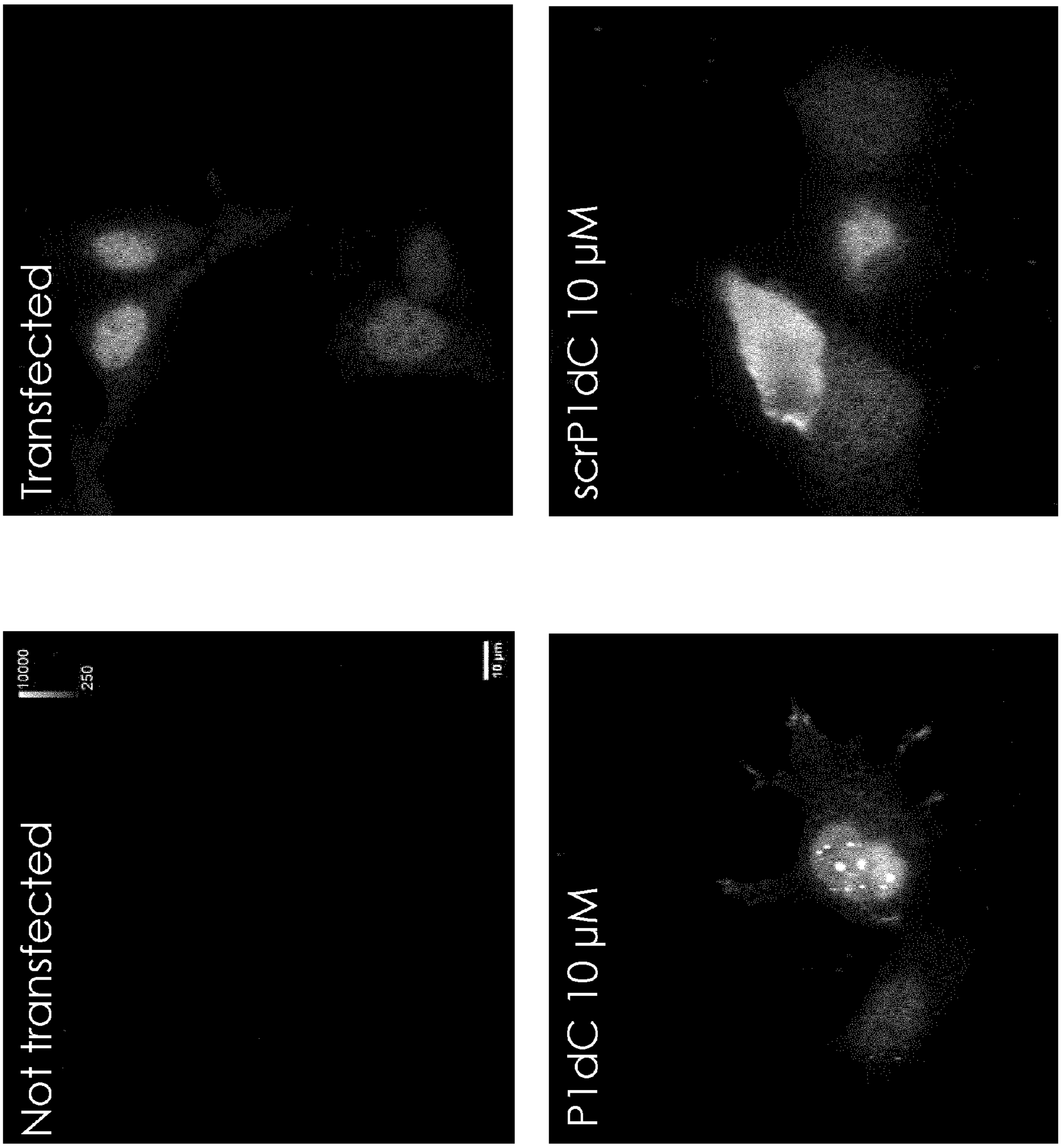
Not transfected
10000
250
10 µm
Transfected
P1dC 10 µM
scrP1dC 10 µM

**Figure 9 A**

1. No Protein
2. mAb16988
3. Anti mouse HRP

1. HBc 550 nM
2. mAb16988
3. Anti mouse HRP

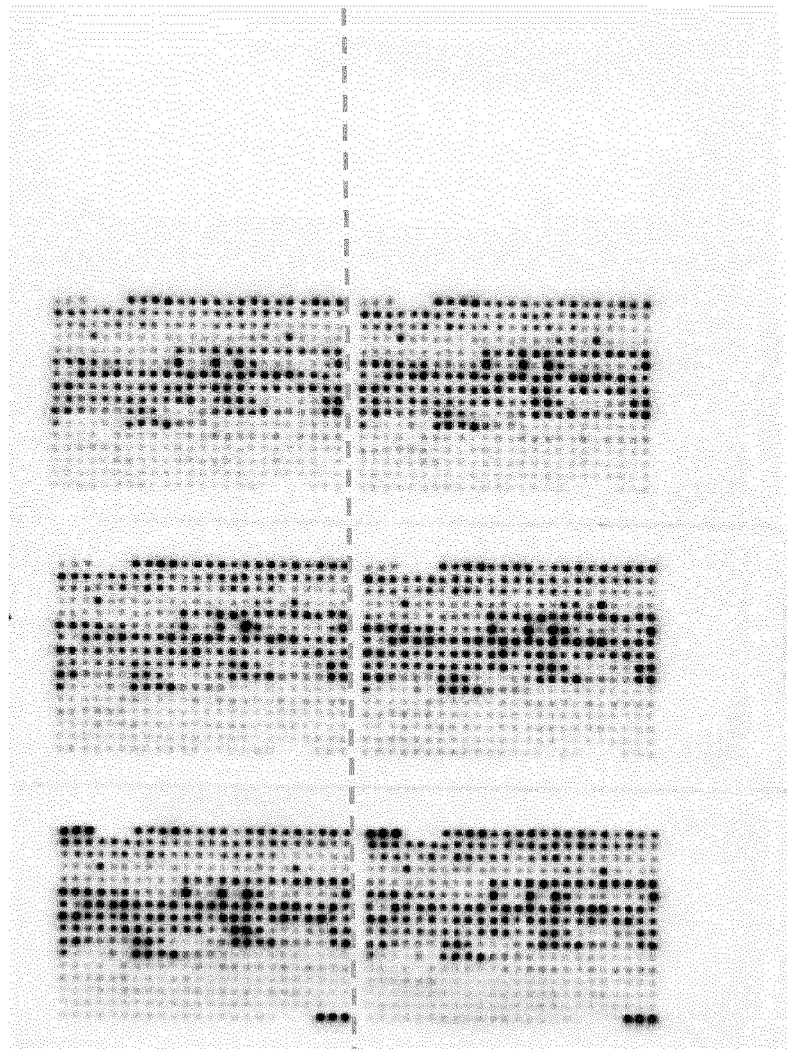

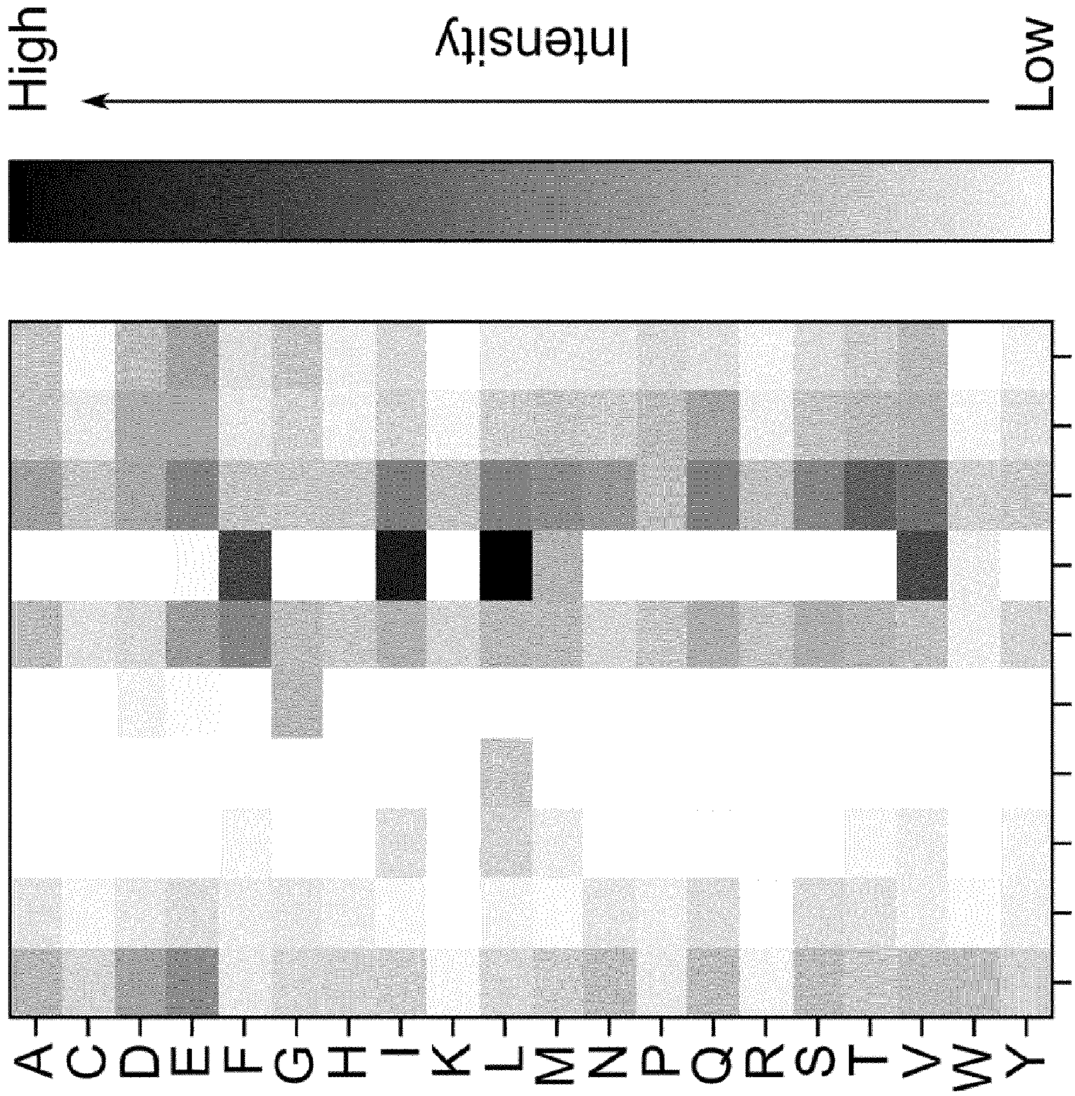


**Figure 9 B**

Europäisches Patentamt
European Patent Office
Office européen des brevets

# EUROPEAN SEARCH REPORT

Application Number
EP 23 15 9984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAKBUL CIHAN ET AL: "Conformational Plasticity of Hepatitis B Core Protein Spikes Promotes Peptide Binding Independent of the Secretion Phenotype", MICROORGANISMS, vol. 9, 956, 29 May 2021 (2021-05-29), page 1-20, XP093064777, ISSN: 2076-2607, DOI: 10.3390/microorganisms9050956 * the whole document * * figure 6; table 1 * | 1-15 | INV. C07K7/06 C07K7/08 C07K14/005 |
| X | PAN X B ET AL: "Artificial recombinant cell-penetrating peptides interfere with envelopment of hepatitis B virus nucleocapsid and viral production", ANTIVIRAL RESEARCH, ELSEVIER BV, NL, vol. 89, no. 1, January 2011 (2011-01), pages 109-114, XP027586733, ISSN: 0166-3542 [retrieved on 2011-01-01] * the whole document * * table 1 * | 1-15 | TECHNICAL FIELDS SEARCHED (IPC) C07K A61K |
| X | WO 00/32625 A1 (BIOGEN INC [US]; MURRAY KENNETH [GB]) 8 June 2000 (2000-06-08) * the whole document * * page 21 - page 25 * | 1-15 | |

The present search report has been drawn up for all claims

3

| Place of search | Date of completion of the search | Examiner |
|---|---|---|
| Munich | 19 July 2023 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 15 9984

This annex lists the patent family members relating to the patent documents cited in the above-mentioned European search report. The members are as contained in the European Patent Office EDP file on
The European Patent Office is in no way liable for these particulars which are merely given for the purpose of information.

19-07-2023

| Patent document cited in search report | Publication date | Patent family member(s) | Publication date |
|---|---|---|---|
| WO 0032625 A1 | 08-06-2000 | AT 525388 T | 15-10-2011 |
| | | AU 770225 B2 | 19-02-2004 |
| | | BR 9915942 A | 21-08-2001 |
| | | CA 2352738 A1 | 08-06-2000 |
| | | CN 1332749 A | 23-01-2002 |
| | | CZ 20011907 A3 | 16-01-2002 |
| | | DK 1135408 T3 | 23-01-2012 |
| | | EA 200100619 A1 | 24-12-2001 |
| | | EE 200100292 A | 15-08-2002 |
| | | EP 1135408 A1 | 26-09-2001 |
| | | EP 2351768 A1 | 03-08-2011 |
| | | EP 2360174 A1 | 24-08-2011 |
| | | HK 1041272 A1 | 05-07-2002 |
| | | HK 1160652 A1 | 10-08-2012 |
| | | HK 1161606 A1 | 27-07-2012 |
| | | HU 0104946 A2 | 29-04-2002 |
| | | IS 5957 A | 31-05-2001 |
| | | JP 2002532387 A | 02-10-2002 |
| | | JP 2008074867 A | 03-04-2008 |
| | | KR 20010081068 A | 25-08-2001 |
| | | NZ 512056 A | 30-01-2004 |
| | | PL 348766 A1 | 03-06-2002 |
| | | SK 7602001 A3 | 05-03-2002 |
| | | TR 200102423 T2 | 21-02-2002 |
| | | US 2002064533 A1 | 30-05-2002 |
| | | US 2003198649 A1 | 23-10-2003 |
| | | US 2004234554 A1 | 25-11-2004 |
| | | US 2007280962 A1 | 06-12-2007 |
| | | US 2012321659 A1 | 20-12-2012 |
| | | WO 0032625 A1 | 08-06-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BLOCK, T. M. ; CHANG, K.-M. ; GUO, J.-T.** Prospects for the Global Elimination of Hepatitis B. *Annual Review of Virology,* 2021, vol. 8 (1), 437-458, https://doi.org/10.1146/annurev-virology-091919-062728
- **BÖTTCHER, B. ; TSUJI, N. ; TAKAHASHI, H. ; DYSON, M. R. ; ZHAO, S. ; CROWTHER, R. A. ; MURRAY, K.** Peptides that block hepatitis B virus assembly: analysis by cryomicroscopy, mutagenesis and transfection. *The EMBO Journal,* 1998, vol. 17 (23), 6839-6845, https://doi.org/10.1093/emboj/17.23.6839
- **CASAÑAL, A. ; LOHKAMP, B. ; EMSLEY, P.** Current developments in Coot for macromolecular model building of Electron Cryo-microscopy and Crystallographic Data. *Protein Science,* 2020, vol. 29 (4), 1055-1064, https://doi.org/10.1002/pro.3791
- **COX, A. L. ; EL-SAYED, M. H. ; KAO, J. H. ; LAZARUS, J. V. ; LEMOINE, M. ; LOK, A. S. ; ZOULIM, F.** Progress towards elimination goals for viral hepatitis. *Nature Reviews Gastroenterology and Hepatology,* 2020, vol. 17 (9), 533-542, https://doi.org/10.1038/s41575-020-0332-6
- **DIKMANS, A. ; BEUTLING, U. ; SCHMEISSER, E. ; THIELE, S. ; FRANK, R.** SC2: A novel process for manufacturing multipurpose high-density chemical microarrays. *QSAR and Combinatorial Science,* 2006, vol. 25 (11), 1069-1080, https://doi.org/10.1002/qsar.200640130
- **DYSON, M. R. ; MURRAY, K.** Selection of peptide inhibitors of interactions involved in complex protein assemblies: association of the core and surface antigens of hepatitis B virus. *Proceedings of the National Academy of Sciences,* 1995, vol. 92 (6), 2194-2198, https://doi.org/10.1073/pnas.92.6.2194
- **GALIBERT, F. ; MANDART, E. ; FITOUSSI, F. ; TIOLLAIS, P. ; CHAMAY, P.** Nucleotide sequence of the hepatitis B virus genome (subtype ayw) cloned in E. coli. *Nature,* 1979, vol. 281 (5733, https://doi.org/10.1038/281646a0
- **GIBSON, D. G.** *Enzymatic Assembly of Overlapping DNA Fragments,* 2011, 349-361, https://doi.org/10.1016/B978-0-12-385120-8.00015-2
- **GRIPON, P. ; CANNIE, I. ; URBAN, S.** Efficient Inhibition of Hepatitis B Virus Infection by Acylated Peptides Derived from the Large Viral Surface Protein. *Journal of Virology,* 2005, vol. 79 (3), 1613-1622, https://doi.org/10.1128/JVI.79.3.1613-1622.2005
- **HU, J. ; PROTZER, U. ; SIDDIQUI, A.** Revisiting Hepatitis B Virus: Challenges of Curative Therapies. *Journal of Virology,* 2019, vol. 93 (20, https://doi.org/10.1128/JVI.01032-19
- **LIEBSCHNER, D. ; AFONINE, P. V. ; BAKER, M. L. ; BUNKÓCZI, G. ; CHEN, V. B. ; CROLL, T. I. ; HINTZE, B. ; HUNG, L.-W. ; JAIN, S. ; MCCOY, A. J.** Macromolecular structure determination using X-rays, neutrons and electrons: recent developments in Phenix. *Acta Crystallographica Section D Structural Biology,* 2019, vol. 75 (10), 861-877, https://doi.org/10.1107/S2059798319011471
- **MAKBUL, C. ; KHAYENKO, V. ; MARIC, H. M. ; BÖTTCHER, B.** Conformational Plasticity of Hepatitis B Core Protein Spikes Promotes Peptide Binding Independent of the Secretion Phenotype. *Microorganisms,* 2021, vol. 9 (5), 956, https://doi.org/10.3390/microorganisms9050956
- **MAKBUL, C. ; KRAFT, C. ; CRRIEßMANN, M. ; RASMUSSEN, T. ; KATZENBERGER, K. ; LAPPE, M. ; PFARR, P. ; STOFFER, C. ; STÖHR, M. ; WANDINGER, A.-M.** Binding of a Pocket Factor to Hepatitis B Virus Capsids Changes the Rotamer Conformation of Phenylalanine 97. *Viruses,* 2021, vol. 13 (11), 2115, https://doi.org/10.3390/v13112115
- **PRISANT, M. G. ; WILLIAMS, C. J. ; CHEN, V. B. ; RICHARDSON, J. S. ; RICHARDSON, D. C.** New tools in MolProbity validation: CaBLAM for CryoEM backbone, UnDowser to rethink "waters," and NGL Viewer to recapture online 3D graphics. *Protein Science,* 2020, vol. 29 (1), 315-329, https://doi.org/10.1002/pro.3786
- **PUNJANI, A. ; ZHANG, H. ; FLEET, D. J.** Non-uniform refinement: adaptive regularization improves single-particle cryo-EM reconstruction. *Nature Methods,* 2020, vol. 17 (12), 1214-1221, https://doi.org/10.1038/s41592-020-00990-8

- **SCHNEIDER, A. F. L. ; BENZ, L. S. ; LEHMANN, M. ; HACKENBERGER, C. P. R.** Cell-Permeable Nanobodies Allow Dual-Color Super-Resolution Microscopy in Untransfected Living Cells. *Angewandte Chemie International Edition,* 2021, vol. 60 (40), 22075-22080, https://doi.org/10.1002/anie.202103068
- **SCHNEIDER, A. F. L. ; KITHIL, M. ; CARDOSO, M. C. ; LEHMANN, M. ; HACKENBERGER, C. P. R.** Cellular uptake of large biomolecules enabled by cell-surface-reactive cell-penetrating peptide additives. *Nature Chemistry,* 2021, vol. 13 (6), 530-539, https://doi. org/10.103 8/s415 57-021-00661-x
- **TIAN, J. ; LI, C. ; LI, W.** Entry of hepatitis B virus: going beyond NTCP to the nucleus. *Current Opinion in Virology,* 2021, vol. 50, 97-102, https://doi.org/10.1016/j.coviro.2021.08.001
- **WATASHI, K. ; WAKITA, T.** Hepatitis B Virus and Hepatitis D Virus Entry, Species Specificity, and Tissue Tropism. *Cold Spring Harbor Perspectives in Medicine,* 2015, vol. 5 (8), a021378, https://doi.org/10.1101/cshperspect.a021378
- *Hepatitis B fact sheet,* 2022, https://www.who.int/en/news-room/fact-sheets/detail/hepatitis-b
- **YANG, Z. ; LASKER, K. ; SCHNEIDMAN-DUHOVNY, D. ; WEBB, B. ; HUANG, C. C. ; PETTERSEN, E. F. ; GODDARD, T. D. ; MENG, E. C. ; SALI, A. ; FERRIN, T. E.** UCSF Chimera, MODELLER, and IMP: An integrated modeling system. *Journal of Structural Biology,* 2012, vol. 179 (3), 269-278, https://doi.org/10.1016/j.jsb.2011.09.006